# EUROPEAN PATENT APPLICATION

(11) **EP 3 978 513 A1**
(43) Date of publication of application: **06.04.2022**
(21) Application number: 20815305.6
(22) Date of filing: 29.05.2020
(51) Int. Cl.: C07K 14/00, C12N 5/10, C12N 1/15, C12N 1/19, C12N 1/21, C12N 15/11, C12N 15/63

(54) **EFFICIENT PPR PROTEIN PRODUCTION METHOD AND USE THEREOF**

(30) Priority: 29.05.2019 JP 2019100551
(71) Applicant: Editforce, Inc., Fukuoka-shi, Fukuoka, 810-0001 (JP); Kyushu University, National University Corporation, Nishi-ku Fukuoka-shi Fukuoka 819-0395 (JP)
(72) Inventor: YAGI,Yusuke, Fukuoka-shi, Fukuoka 810-0001 (JP); NAKAMURA, Takahiro, Fukuoka-shi, Fukuoka 819-0395 (JP)
(74) Representative: Hoffmann Eitle
(86) International application number: PCT/JP2020/021472
(87) International publication number: WO 2020/241876

(57) **Abstract**

A PPR protein with high performance is provided. A PPR protein that binds to a long nucleotide sequence is provided by linking motifs in a number larger than conventionally used 7 to 14. A PPR motif is provided, of which typical examples are the followings: (A-1) a PPR motif consisting of the sequence of SEQ ID NO: 9 or 401, (C-1) a PPR motif consisting of the sequence of SEQ ID NO: 10, (G-1) a PPR motif consisting of the sequence of SEQ ID NO: 11, and (U-1) a PPR motif consisting of the sequence of SEQ ID NO: 12. These motifs are useful as PPR motifs for adenine, cytosine, guanine, and uracil in a target nucleotide sequence, respectively.

## Description

### Technical Field

The present invention relates to a nucleic acid manipulation technique using a protein capable of binding to a target nucleic acid. The present invention is useful in a wide range of fields, including medicine (drug discovery support, therapeutic treatment etc.), agriculture (agricultural, fishery and livestock production, breeding etc.), and chemistry (biological material production etc.).

### Background Techniques

PPR proteins are proteins comprising repeat of PPR motifs each having about 35 amino acids length, and one PPR motif can specifically bind to one base. The combination of the first, fourth, and ii-th (second from the end before the next motif) amino acids in a PPR motif determines to which one of adenine, cytosine, guanine, and uracil (or thymine) the motif binds (Patent documents 1 and 2).

Since the PPR motifs attain the binding by recognizing a single base with one motif, when designing, for example, a PPR protein that specifically binds to an 18-base long nucleic acid sequence, 18 PPR motifs should be linked together. So far, artificial PPR proteins comprising 7 to 14 PPR motifs linked together have been reported (Non-patent documents 1 to 6).

### Prior Art References

### Patent documents

Patent document 1: International Publication WO2013/058404
Patent document 2: International Publication WO2014/175284

### Non-patent documents

Non-patent document 1: Coquille, S. et al., An artificial PPR scaffold for programmable RNA recognition, Nature Communications 5, Article number: 5729 (2014)
Non-patent document 2: Shen, C. et al., Specific RNA Recognition by Designer Pentatricopeptide Repeat Protein, Molecular Plant 8, 667-670 (2015)
Non-patent document 3: Shen, C. et al., Structural basis for specific single-stranded RNA recognition by designer pentatricopeptide repeat proteins, Nature Communications, Volume 7, Article number: 11285 (2016)
Non-patent document 4: Gully, B.S. et al., The design and structural characterization of a synthetic pentatricopeptide repeat protein, Acta Cryst., D71, 196-208 (2015)
Non-patent document 5: Miranda, R.G. et al., RNA-binding specificity landscapes of designer pentatricopeptide repeat proteins elucidate principles of PPR-RNA interactions, Nucleic Acids Research, 46(5), 2613-2623 (2018)
Non-patent document 6: Yan, J. et al., Delineation of pentatricopeptide repeat codes for target RNA prediction, Nucleic Acids Research, gkz075 (2019)

### Summary of the Invention

### Object to be achieved by the invention

High performance PPR proteins are required in order that the PPR proteins specifically bind to a target RNA molecule in cells, and manipulations can be performed with them as wanted.

In addition, in order that the PPR proteins specifically bind to a target RNA molecule in cells, and manipulations can be performed with them as wanted, PPR proteins that comprise linked motifs more than 7 to 14 conventionally used and can bind to longer sequences are required. For example, the human genomes comprise 6 billion base pairs constituted by the four kinds of bases (A, C, G, and T or U), and therefore a sequence of at least 17 nucleotides is required to specify a single nucleotide sequence from the sequences of the genomes (this is because 4¹⁶ is 4 billions, and 4¹⁷ is 16 billions).

### Means for achieving the object

The present invention provides the followings as novel PPR motifs and so forth.
[1] A PPR motif, which is any one of the following PPR motifs:
   (A-1) a PPR motif consisting of the sequence of SEQ ID NO: 9, or a PPR motif consisting of the sequence of SEQ ID NO: 9 having a substitution selected from the group consisting of substitution of the amino acid at position 10 with tyrosine, substitution of the amino acid at position 15 with lysine, substitution of the amino acid at position 16 with leucine, substitution of the amino acid at position 17 with glutamic acid, substitution of the amino acid at position 18 with aspartic acid, and substitution of the amino acid at position 28 with glutamic acid, or a PPR motif consisting of the sequence of SEQ ID NO: 401 or a PPR motif consisting of the sequence of SEQ ID NO: 401 having a substitution selected from the group consisting of substitution of the amino acid at position 10 with tyrosine, substitution of the amino acid at position 16 with leucine, substitution of the amino acid at position 17 with glutamic acid, substitution of the amino acid at position 18 with aspartic acid, and substitution of the amino acid at position 28 with glutamic acid;
   (A-2) a PPR motif consisting of the sequence of SEQ ID NO: 9 or 401 having a substitution, deletion, or addition of 1 to 20 amino acids other than the amino acids at positions 1, 2, 3, 4, 6, 7, 9, 11, 12, 14, 19, 26, 30, 33, and 34, and having an adenine-binding property;
   (A-3) a PPR motif having a sequence identity of at least 42% to the sequence of SEQ ID NO: 9 or 401, provided that the amino acids at positions 1, 2, 3, 4, 6, 7, 9, 11, 12, 14, 19, 26, 30, 33, and 34 are identical, and having an adenine-binding property;
   (C-1) a PPR motif consisting of the sequence of SEQ ID NO: 10, or a PPR motif consisting of the sequence of SEQ ID NO: 10 having a substitution of amino acid selected from the group consisting of substitution of the amino acid at position 2 with serine, substitution of the amino acid at position 5 with isoleucine, substitution of the amino acid at position 7 with leucine, substitution of the amino acid at position 8 with lysine, substitution of the amino acid at position 10 with phenylalanine or tyrosine, substitution of the amino acid at position 15 with arginine, substitution of the amino acid at position 22 with valine, substitution of the amino acid at position 24 with arginine, substitution of the amino acid at position 27 with leucine, and substitution of the amino acid at position 29 with arginine;
   (C-2) a PPR motif consisting of the sequence of SEQ ID NO: 10 having a substitution, deletion, or addition of 1 to 25 amino acids other than the amino acids at positions 1, 3, 4, 14, 18, 19, 26, 30, 33, and 34, and having a cytosine-binding property;
   (C-3) a PPR motif having a sequence identity of at least 25% to the sequence of SEQ ID NO: 10, provided that the amino acids at positions 1, 3, 4, 14, 18, 19, 26, 30, 33, and 34 are identical, and having a cytosine-binding property;
   (G-1) a PPR motif consisting of the sequence of SEQ ID NO: 11, or a PPR motif consisting of the sequence of SEQ ID NO: 11 having a substitution selected from the group consisting of substitution of the amino acid at position 10 with phenylalanine, substitution of the amino acid at position 15 with aspartic acid, substitution of the amino acid at position 27 with valine, substitution of the amino acid at position 28 with serine, and substitution of the amino acid at position 35 with isoleucine;
   (G-2) a PPR motif consisting of the sequence of SEQ ID NO: 11 having a substitution, deletion, or addition of 1 to 21 amino acids other than the amino acids at positions 1, 2, 3, 4, 6, 7, 9, 14, 18, 19, 26, 30, 33, and 34, and having a guanine-binding property; (G-3) a PPR motif having a sequence identity of at least 40% to the sequence of SEQ ID NO: 11, provided that the amino acids at positions 1, 2, 3, 4, 6, 7, 9, 14, 18, 19, 26, 30, 33, and 34 are identical, and having a guanine-binding property;
   (U-1) a PPR motif consisting of the sequence of SEQ ID NO: 12, or a PPR motif consisting of the sequence of SEQ ID NO: 12 having a substitution selected from the group consisting of substitution of the amino acid at position 10 with phenylalanine, substitution of the amino acid at position 13 with serine, substitution of the amino acid at position 15 with lysine, substitution of the amino acid at position 17 with glutamic acid, substitution of the amino acid at position 20 with leucine, substitution of the amino acid at position 21 with lysine, substitution of the amino acid at position 23 with phenylalanine, substitution of the amino acid at position 24 with aspartic acid, substitution of the amino acid at position 27 with lysine, substitution of the amino acid at position 28 with lysine, substitution of the amino acid at position 29 with arginine, and substitution of the amino acid at position 31 with leucine;
   (U-2) a PPR motif consisting of the sequence of SEQ ID NO: 12 having a substitution, deletion, or addition of 1 to 22 amino acids other than the amino acids at positions 1, 2, 3, 4, 6, 11, 12, 14, 19, 26, 30, 33, and 34, and having a uracil-binding property; and (U-3) a PPR motif having a sequence identity of at least 37% to the sequence of SEQ ID NO: 12, provided that the amino acids at positions 1, 2, 3, 4, 6, 11, 12, 14, 19, 26, 30, 33, and 34 are identical, and having a uracil-binding property.
[2] Use of the PPR motif according to 1 for preparation of a PPR protein of which target RNA has a length of 15 bases or longer.
[3] Use of the PPR motif according to 1 for preparation of a PPR protein, which is for enhancing binding performance of the PPR protein to a target RNA.
[4] A PPR protein comprising n of PPR motifs and capable of binding to a target RNA consisting of a sequence of n bases in length , wherein:
   the PPR motif for adenine in the base sequence is the PPR motif of (A-1), (A-2), or (A-3) defined in 1;
   the PPR motif for cytosine in the base sequence is the PPR motif of (C-1), (C-2), or (c-3) defined in 1;
   the PPR motif for guanine in the base sequence is the PPR motif of (G-1), (G-2), or (G-3) defined in 1; and
   the PPR motif for uracil in the base sequence is the PPR motif of (U-1), (U-2), or (U-3) defined in 1.
[5] The protein according to 4, wherein n is 15 or larger.
[6] The protein according to 4 or 5, wherein the first PPR motif from the N-terminus is any one of the following motifs:
   (1st A-1) a PPR motif consisting of the sequence of SEQ ID NO: 402 having such substitutions of the amino acids at positions 6 and 9 that any one of the combinations defined below is satisfied;
   (1st_A-2) a PPR motif consisting of the sequence of (1st_A-1) having a substitution, deletion, or addition of 1 to 9 amino acids other than the amino acids at positions 1, 4, 6, 9, and 34, and having an adenine-binding property;
   (1st_A-3) a PPR motif having a sequence identity of at least 80% to the sequence of (1st_A-1), provided that the amino acids at positions 1, 4, 6, 9, and 34 are identical, and having an adenine-binding property;
   (1st_C-1) a PPR motif consisting of the sequence of SEQ ID NO: 403;
   (1st_C-2) a PPR motif comprising the sequence of (1st_C-1) having a substitution, deletion, or addition of 1 to 9 amino acids other than the amino acids at positions 1, 4, 6, 9, and 34, and having a cytosine-binding property;
   (1st_C-3) a PPR motif having a sequence identity of at least 80% to the sequence of (1st_C-1), provided that the amino acids at positions 1, 4, 6, 9, and 34 are identical, and having a cytosine binding property;
   (1st_G-1) a PPR motif consisting of the sequence of SEQ ID NO: 404 having such substitutions of the amino acids at positions 6 and 9 that any one of the combinations defined below is satisfied;
   1lst_G-2) a PPR motif comprising the sequence of (1st_G-1) having a substitution, deletion, or addition of 1 to 9 amino acids other than the amino acids at positions 1, 4, 6, 9, and 34, and having a guanine-binding property;
   (1st_G-3) a PPR motif having a sequence identity of at least 80% to the sequence of (1st_G-1), provided that the amino acids at positions 1, 4, 6, 9, and 34 are identical, and having a guanine-binding property;
   (1st_U-1) a PPR motif consisting of the sequence of SEQ ID NO: 405 having such substitutions of the amino acids at positions 6 and 9 that any one of the combinations defined below is satisfied;
   (1st_U-2) a PPR motif comprising the sequence of (1st_U-1) having a substitution, deletion, or addition of 1 to 9 amino acids other than the amino acids at positions 1, 4, 6, 9, and 34, and having a uracil-binding property; and
   (1st_U-3) a PPR motif having a sequence identity of at least 80% to the sequence of (1st_U-1), provided that the amino acids at positions 1, 4, 6, 9, and 34 are identical,
   and having a uracil-binding property:
      - a combination of asparagine as the amino acid at position 6 and glutamic acid as the amino acid at position 9,
      - a combination of asparagine as the amino acid at position 6 and glutamine as the amino acid at position 9,
      - a combination of asparagine as the amino acid at position 6 and lysine as the amino acid at position 9, and
      - a combination of aspartic acid as the amino acid at position 6 and glycine as the amino acid at position 9.
[7] A method for controlling RNA splicing, which uses the protein according to any one of 4 to 6.
[8] A method for detecting RNA, which uses the protein according to any one of 4 to 6.
[9] A fusion protein of at least one selected from the group consisting of a fluorescent protein, a nuclear localization signal peptide, and a tag protein, and the protein according to any one of 4 to 6.
[10] A nucleic acid encoding the PPR motif according to 1, or the protein according to any one of 4 to 6.
[11] A vector comprising the nucleic acid according to 10.
[12] A cell (except for human individual) containing the vector according to 11.
[13] A method for manipulating RNA, which uses the PPR motif according to 1, the protein according to any one of 4 to 6, or the vector according to 11 (implementation in human individual is excluded).
[14] A method for producing an organism, which comprises the manipulation method according to 13.

[1] A PPR motif, which is any one of the following PPR motifs:
   (A-1) a PPR motif consisting of the sequence of SEQ ID NO: 9, or a PPR motif consisting of the sequence of SEQ ID NO: 9 having a substitution selected from the group consisting of substitution of the amino acid at position 10 with tyrosine, substitution of the amino acid at position 15 with lysine, substitution of the amino acid at position 16 with leucine, substitution of the amino acid at position 17 with glutamic acid, substitution of the amino acid at position 18 with aspartic acid, and a substitution of the amino acid at position 28 with glutamic acid;
   (A-2) a PPR motif consisting of the sequence of SEQ ID NO: 9 having a substitution, deletion, or addition of 1 to 20 amino acids other than the amino acids at positions 1, 2, 3, 4, 6, 7, 9, 11, 12, 14, 19, 26, 30, 33, and 34, and having an adenine-binding property;
   (A-3) a PPR motif having a sequence identity of at least 42% to the sequence of SEQ ID NO: 9, provided that the amino acids at positions 1, 2, 3, 4, 6, 7, 9, 11, 12, 14, 19, 26, 30, 33, and 34 are identical, and having an adenine-binding property;
   (C-1) a PPR motif consisting of the sequence of SEQ ID NO: 10, or a PPR motif consisting of the sequence of SEQ ID NO: 10 having a substitution of amino acid selected from the group consisting of substitution of the amino acid at position 2 with serine, substitution of the amino acid at position 5 with isoleucine, substitution of the amino acid at position 7 with leucine, substitution of the amino acid at position 8 with lysine, substitution of the amino acid at position 10 with phenylalanine or tyrosine, substitution of the amino acid at position 15 with arginine, substitution of the amino acid at position 22 with valine, substitution of the amino acid at position 24 with arginine, substitution of the amino acid at position 27 with leucine, and substitution of the amino acid at position 29 with arginine;
   (C-2) a PPR motif consisting of the sequence of SEQ ID NO: 10 having a substitution, deletion, or addition of 1 to 25 amino acids other than the amino acids at positions 1, 3, 4, 14, 18, 19, 26, 30, 33, and 34, and having a cytosine-binding property;
   (C-3) a PPR motif having a sequence identity of at least 25% to the sequence of SEQ ID NO: 10, provided that the amino acids at positions 1, 3, 4, 14, 18, 19, 26, 30, 33, and 34 are identical, and having a cytosine-binding property;
   (G-1) a PPR motif consisting of the sequence of SEQ ID NO: 11, or a PPR motif consisting of the sequence of SEQ ID NO: 11 having a substitution selected from the group consisting of substitution of the amino acid at position 10 with phenylalanine, substitution of the amino acid at position 15 with aspartic acid, substitution of the amino acid at position 27 with valine, substitution of the amino acid at position 28 with serine, and substitution of the amino acid at position 35 with isoleucine;
   (G-2) a PPR motif consisting of the sequence of SEQ ID NO: 11 having a substitution, deletion, or addition of 1 to 21 amino acids other than the amino acids at positions 1, 2, 3, 4, 6, 7, 9, 14, 18, 19, 26, 30, 33, and 34, and having a guanine-binding property;
   (G-3) a PPR motif having a sequence identity of at least 40% to the sequence of SEQ ID NO: 11, provided that the amino acids at positions 1, 2, 3, 4, 6, 7, 9, 14, 18, 19, 26, 30, 33, and 34 are identical, and having a guanine-binding property;
   (U-1) a PPR motif consisting of the sequence of SEQ ID NO: 12, or a PPR motif consisting of the sequence of SEQ ID NO: 12 having a substitution selected from the group consisting of substitution of the amino acid at position 10 with phenylalanine, substitution of the amino acid at position 13 with serine, substitution of the amino acid at position 15 with lysine, substitution of the amino acid at position 17 with glutamic acid, substitution of the amino acid at position 20 with leucine, substitution of the amino acid at position 21 with lysine, substitution of the amino acid at position 23 with phenylalanine, substitution of the amino acid at position 24 with aspartic acid, substitution of the amino acid at position 27 with lysine, substitution of the amino acid at position 28 with lysine, substitution of the amino acid at position 29 with arginine, and substitution of the amino acid at position 31 with leucine;
   (U-2) a PPR motif consisting of the sequence of SEQ ID NO: 12 having a substitution, deletion, or addition of 1 to 22 amino acids other than the amino acids at positions 1, 2, 3, 4, 6, 11, 12, 14, 19, 26, 30, 33, and 34, and having a uracil-binding property; and
   (U-3) a PPR motif having a sequence identity of at least 37% to the sequence of SEQ ID NO: 12, provided that the amino acids at positions 1, 2, 3, 4, 6, 11, 12, 14, 19, 26, 30, 33, and 34 are identical, and having a uracil-binding property.
[2] Use of the PPR motif according to 1 for preparation of a PPR protein of which target RNA has a length of 15 bases or longer.
[3] Use of the PPR motif according to 1 for preparation of a PPR protein, which is for enhancing binding performance of the PPR protein to a target RNA.
[4] A protein comprising n of PPR motifs and capable of binding to a target RNA consisting of a sequence of n bases in length, wherein:
   the PPR motif for adenine in the base sequence is the PPR motif of (A-1), (A-2), or (A-3) defined in 1;
   the PPR motif for cytosine in the base sequence is the PPR motif of (C-1), (C-2), or (c-3) defined in 1;
   the PPR motif for guanine in the base sequence is the PPR motif of (G-1), (G-2), or (G-3) defined in 1; and
   the PPR motif for uracil in the base sequence is the PPR motif of (U-1), (U-2), or (U-3) defined in 1.
[5] The protein according to 4, wherein n is 15 or larger.
[6] A method for controlling RNA splicing, which uses the protein according to 4 or 5.
[7] A method for detecting RNA, which uses the protein according to 4 or 5.
[8] A fusion protein of at least one selected from the group consisting of a fluorescent protein, a nuclear localization signal peptide, and a tag protein, and the protein according to 4 or 5.
[9] A nucleic acid encoding the PPR motif according to 1, or the protein according to 4 or 5.
[10] A vector comprising the nucleic acid according to 9.
[11] A cell (except for human individual) containing the vector according to 10.
[12] A method for manipulating RNA, which uses the PPR motif according to 1, the protein according to 4 or 5, or the vector according to 10 (implementation in human individual is excluded).
[13] A method for producing an organism, which comprises the manipulation method according to 12.
[14] A method for preparing a gene encoding a protein comprising n of PPR motifs that can bind to a target nucleic acid consisting of a sequence of n bases in length, which comprises the following steps:
   selecting m kinds of PPR parts required to prepare the objective gene from a library of at least 20 x m kinds of PPR parts, which consist of at least m kinds of intermediate vectors Dest-a, ... , which are designed so that they can successively linked, and are each inserted with at least 20 kinds of polynucleotides including 4 kinds encoding PPR motifs that have adenine, cytosine-, guanine-, and uracil- or thymine-binding properties, respectively, and 16 kinds encoding linkage products of two of the PPR motifs, respectively; and
   subjecting the selected m kinds of PPR parts to the Golden Gate reaction together with vector parts to obtain a vector in which m of polynucleotide linkage products are inserted (where n is m or larger, and is m x 2 or smaller).
[15] The preparation method according to 14, wherein m is 10, and which is for preparing a gene encoding a protein containing 15 or more of PPR motifs.
[16] A method for detecting or quantifying a protein comprising n of PPR motifs that can bind to a target nucleic acid consisting of a sequence of n bases in length, which comprises the following step:
   the step of adding a solution containing a candidate protein to a solid-phased target nucleic acid, and detecting or quantifying the protein that bound to the target nucleic acid.
[17] The method according to 16, wherein the candidate protein is fused to a marker protein.

### Brief Description of the Drawings

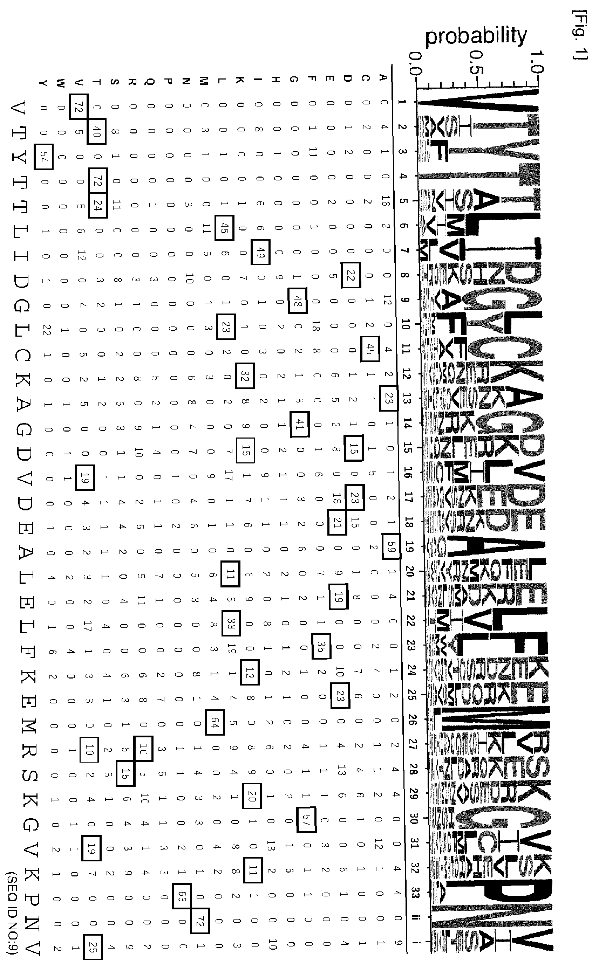

[Fig. 1] v2 Motif (that recognizes adenine)

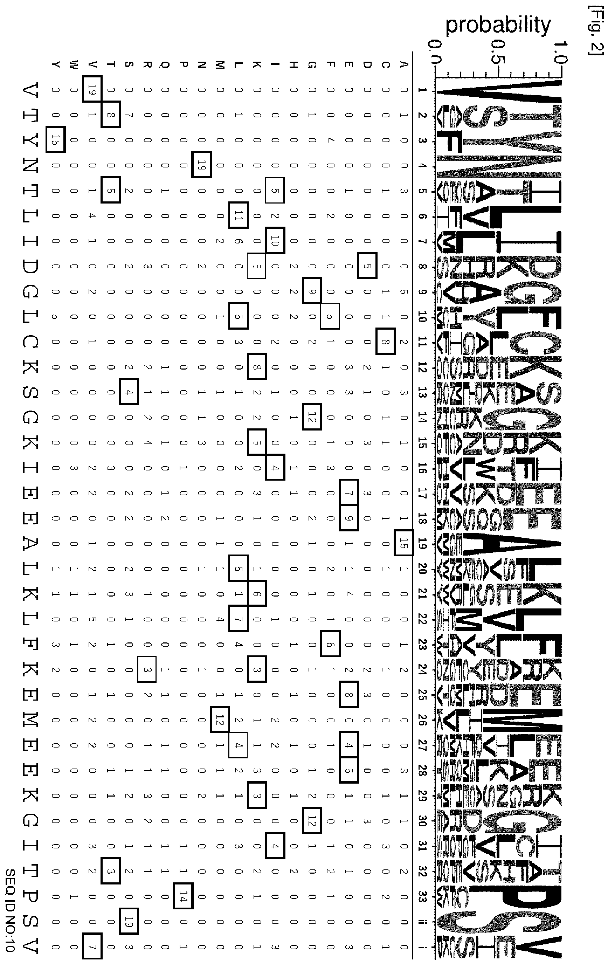

[Fig. 2] v2 Motif (that recognizes cytosine)

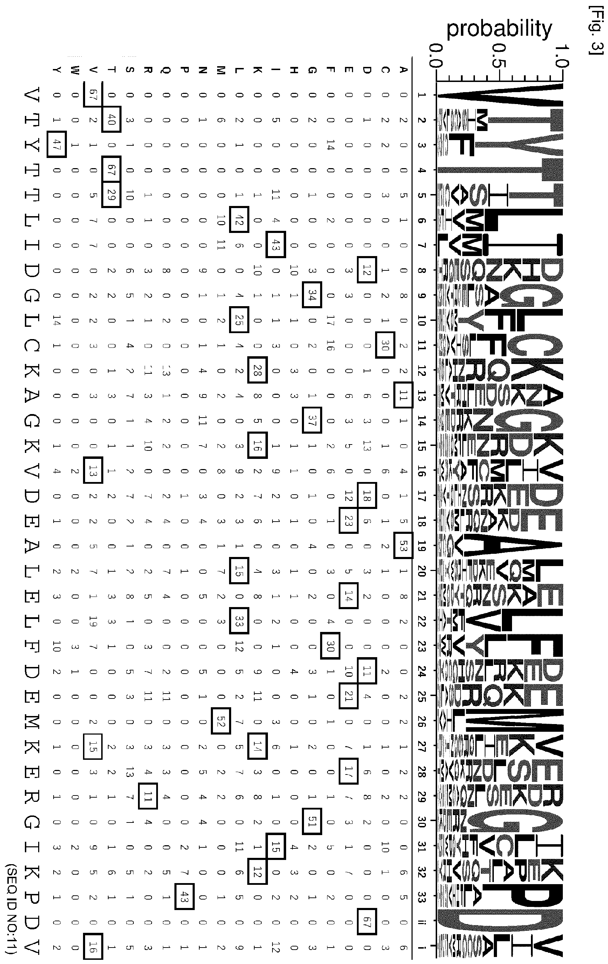

[Fig. 3] v2 Motif (that recognizes guanine)

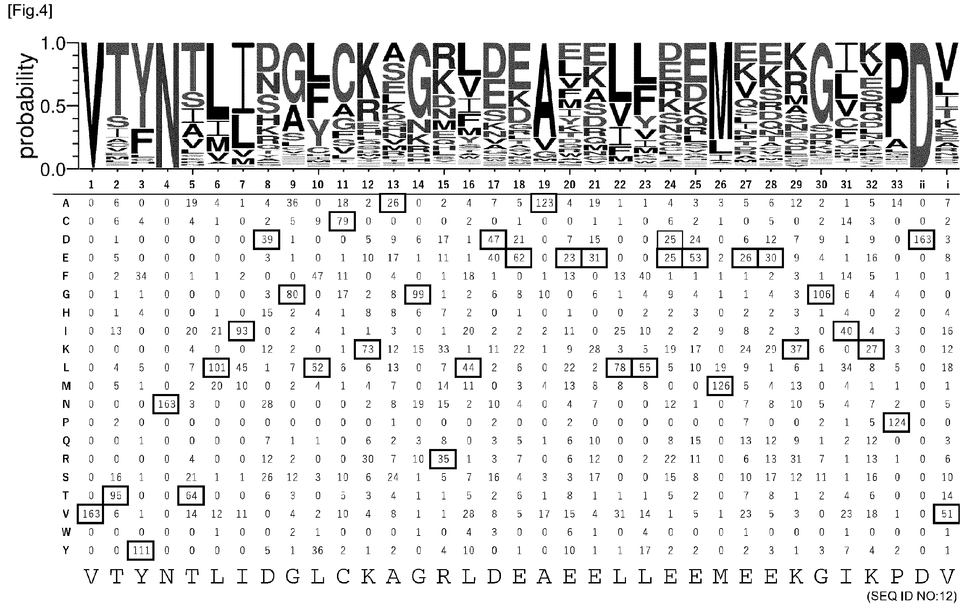

[Fig. 4] v2 Motif (that recognizes uracil)

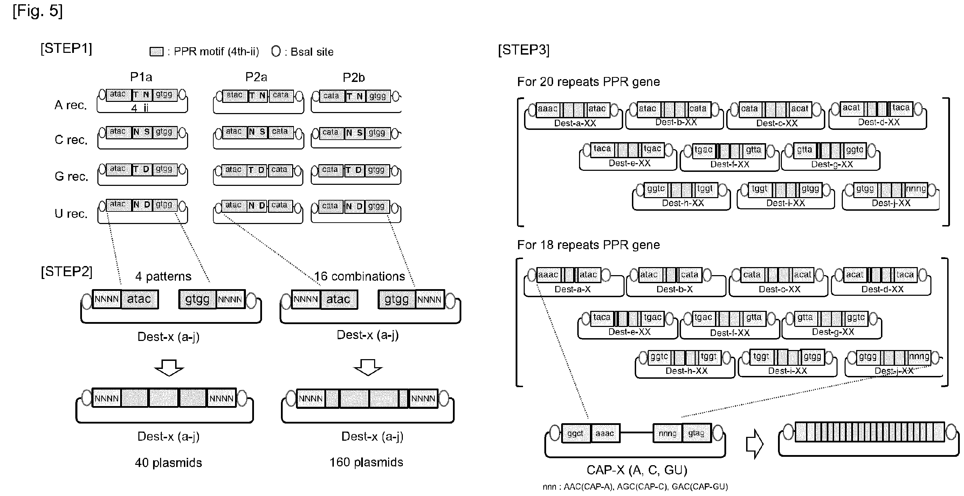

[Fig. 5] An example of the cloning method for seamlessly linking PPR motif sequences.

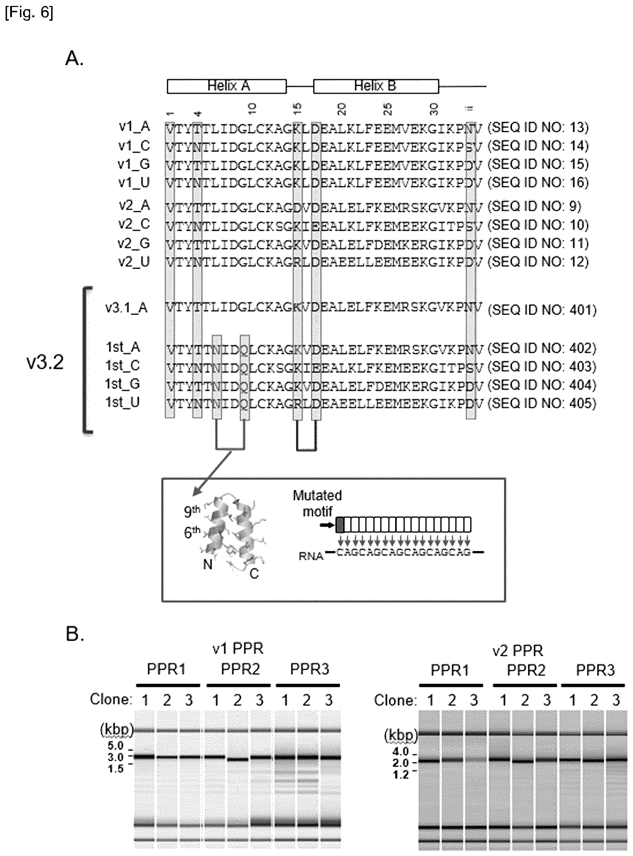

[Fig. 6] Verification of seamless cloning using libraries of PPR motifs comprising 1 or 2 motifs. A: The amino acid sequences of v1, v2, v3.1, and v3.2 motifs. v3.1 corresponds to v2 introduced with a D15K mutation in the adenine recognition motif. For v3.2 motif, 1st_x is chosen for the first motif, and the second and following motifs are selected from v2_C, v2_G, v2_U, and v3.1_A. B: The results of the preparation of three clones for each of the three kinds of 18-motif PPR proteins. With v1, correct size bands were obtained except for the second clone of PPR2. With v2, correct size bands were obtained for all clones.

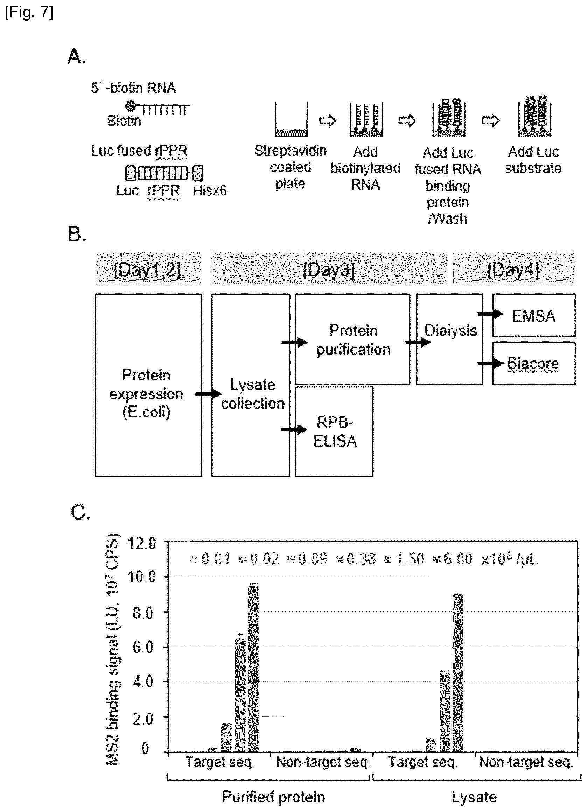

[Fig. 7] An example of high throughput evaluation of binding performance of RNA-binding proteins. A: Comparison of typical nucleic acid-protein binding experiment schemes. B: Outline of RPB-ELISA (RNA-protein binding ELISA). C: Experimental results obtained with MS2 protein and a target RNA thereof. Specific bindings were detected with both the purified protein solution (Purified protein) and *E*. *coli* lysate (Lysate).

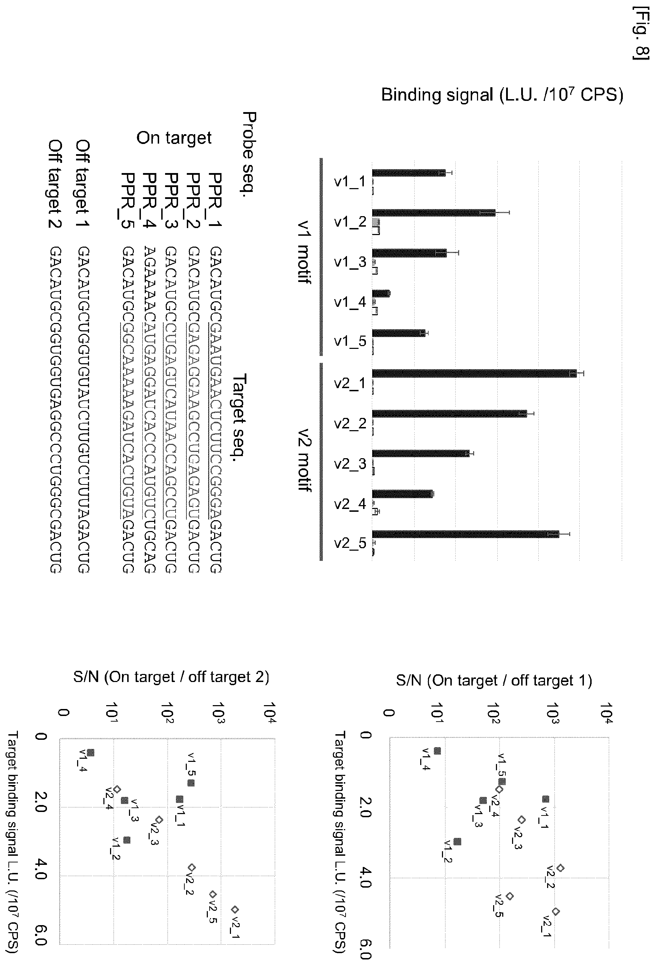

[Fig. 8] The results of RNA binding performance comparison experiments. When the proteins were prepared with the motif sequence v2, there was observed increase (1.3 to 3.6-fold) in the binding power to the target sequence for all the proteins compared with the proteins prepared with the motif sequence v1. In addition, higher target binding signal/non-target binding signal (S/N) was obtained with v2 for all the proteins compared with the proteins obtained with v1, indicating that v2 provides higher affinity and specificity for the target compared with v1. In the upper left graph (Binding signal (L.U./10⁷ CPS)), black bars show the results for RNA probes having target sequences, gray bars show the results for RNA probes having Off target 1, and white bars show the results for RNA probes having Off target 2. In the probe sequences (Prob seq.) shown in the lower left part, the underlines indicate the target sequences (Target seq.).

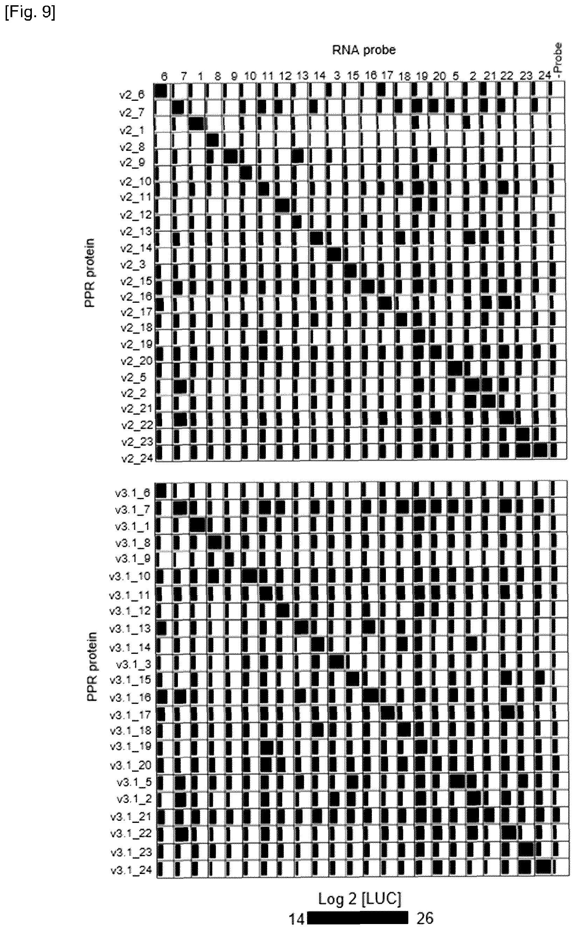

[Fig. 9] Detailed analysis of RNA binding performance (specificity) of the PPR proteins. PPR proteins for 23 kinds of target sequences were prepared by using the v2 motif, and all binding combinations were analyzed by using RPB-ELISA. It was found that 21 kinds of PPR proteins showed the strongest binding power to their targets (upper part). Similarly, the RNA binding performance was analyzed by using the V3.1 motif (lower part).

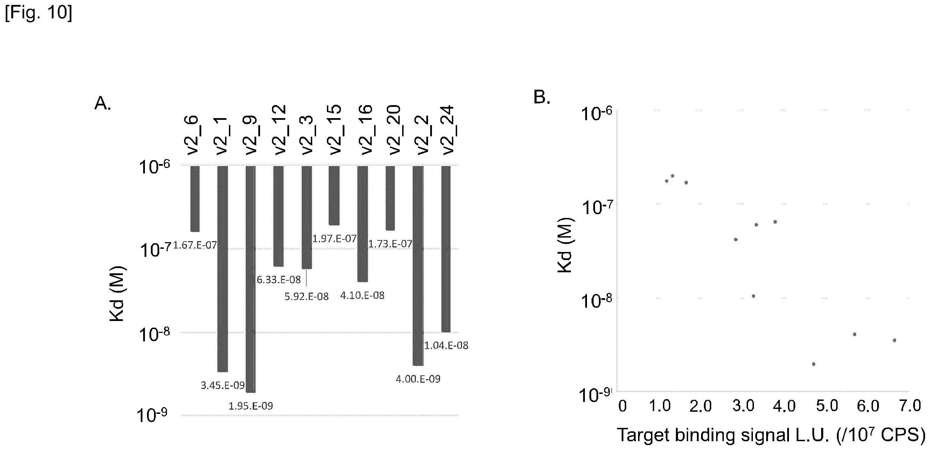

[Fig. 10] Detailed analysis of RNA binding performance (affinity) of the PPR proteins. A: Kd values of the prepared proteins for their targets. The minimum value was 1.95 × 10⁻⁹, which is the lowest Kd value among those of the designed PPR proteins reported so far. B: Correlation between the Kd value and the signal value obtained from the binding experiment using RPB-ELISA. It can be estimated that when the luminescence value observed in RPB-ELISA is 1.0 to 2.0 × 10⁷, the Kd value is 10⁻⁶ to 10⁻⁷ M; when the luminescence value observed in RPB-ELISA is 2.0 to 4.0 × 10⁷, the Kd value is 10⁻⁷ to 10⁻⁸ M; and when the luminescence value observed in RPB-ELISA is higher than 4.0 × 10⁷, the Kd value is ~10⁻⁸ or lower.

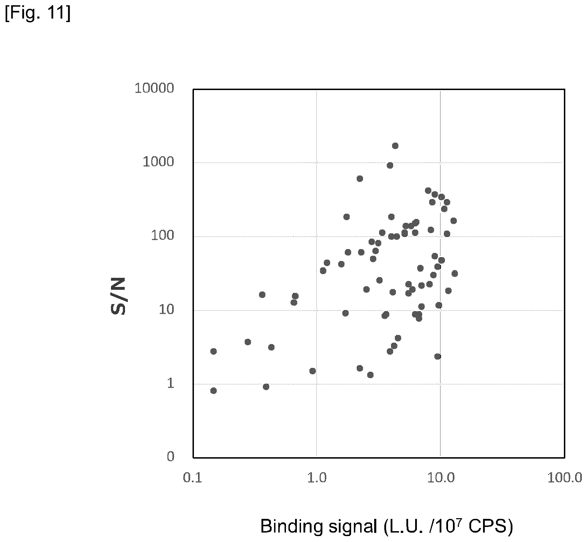

[Fig. 11] Successful construction probability. PPR proteins for 72 kinds of target sequences were prepared by using the v2 motif, and probability of successful construction was calculated by using RPB-ELISA. Among the 72 kinds of the PPR proteins, 63 kinds (88%) were estimated to have a Kd value of 10⁻⁸ M or lower (RPB-ELISA value is higher than 1 × 10⁷). Further, 54 (75%) of them had a specificity value (S/N) higher than 10, which value is for evaluation of the specificity, and is calculated by dividing the target binding signal with the non-target binding signal. These results indicate that by preparing the PPR protein using the v2 motif, sequence-specific RNA-binding proteins can be efficiently prepared.

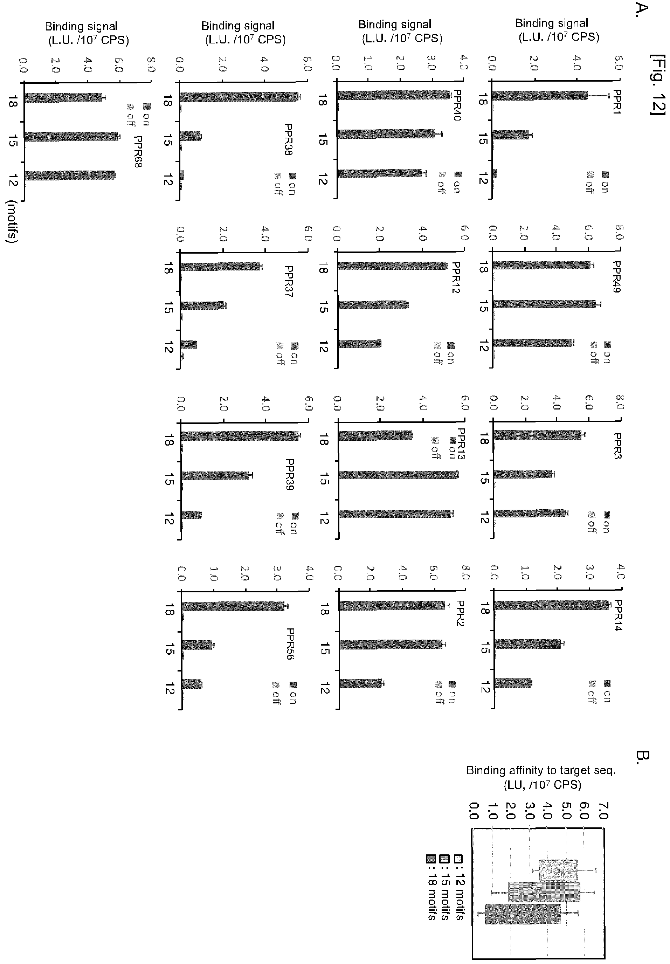

[Fig. 12] Evaluation of target binding activity in relation with the number of PPR motifs. A: Results for the respective target sequences. B: Averages of the values for those of 18, 15, and 12 motifs. It was found that a larger number of motifs provides higher binding strength, and when those of 18 motifs and 15 motifs are compared, a protein with high binding strength can be stably prepared with 18 motifs.

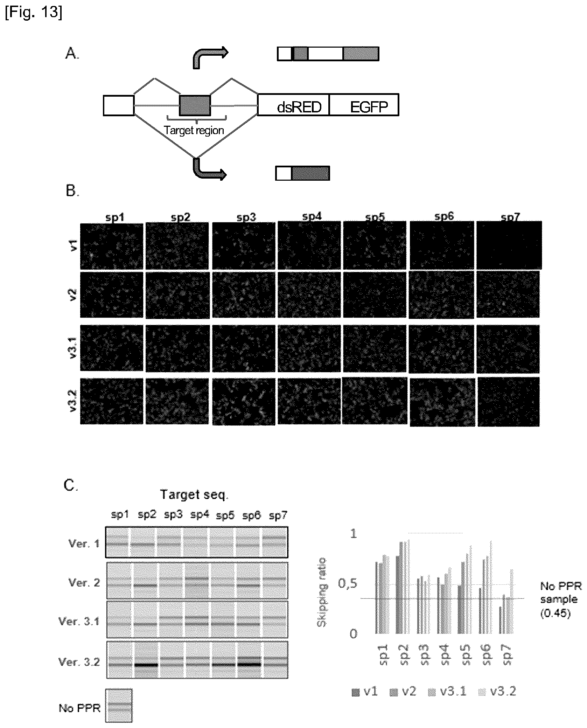

[Fig. 13] An example of artificial control of splicing with PPR proteins. A: Experimental scheme. Sequences of 18 nucleotides were chosen from the regions of intron 1, exon 2, and intron 2, and an experiment was performed for determining whether the amount ratio of the splicing variants of the RG-6 reporter could be changed depending on the PPR proteins binding to the sequences. B: GFP and RFP fluorescence images of cells obtained after cultured with the PPR expression plasmid DNA and RG-6 reporter plasmid DNA. C: Splicing variant ratio. Total RNA was extracted from the cells after the fluorescence images were taken, and the amplification products of RT-PCR were electrophoresed. Intensities of the band of about 114 bp, which was regarded as band of PCR product (a) obtained with exon skipping, and the band of about 142 bp, which was regarded as band of PCR product (b) obtained without skipping, were measured. The splicing ratio was calculated as a/(a + b). It was found that the splicing ratio was significantly changed by introduction of PPR. It was verified that exon skipping can be changed by using the PPR proteins, and in addition, it was found that splicing can be more efficiently changed by using the v2 motif.

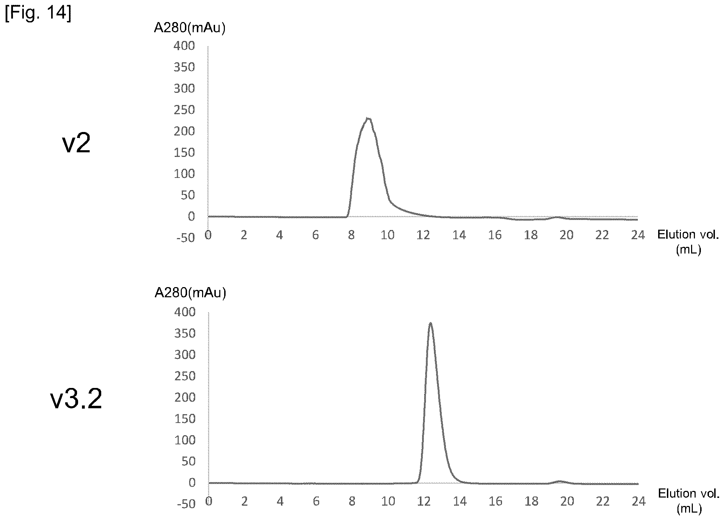

[Fig. 14] Effect of the first PPR motif from the N-terminus on aggregation. Each PPR protein was prepared in an *Escherichia coli* (*E. coli*) expression system, purified, and separated by gel filtration chromatography. A smaller volume of the elution fraction (Elution vol.) indicates a larger molecular size. Those using v2 were eluted in elution fractions of 8 to 10 mL, while the elution peak was observed for elution fractions of 12 to 14 mL with v3.2. This suggested a possibility of aggregation of the proteins due to the larger protein size obtained with v2, and it was found that the aggregation was improved with v3.2.

### Modes for Carrying out the Invention

### [PPR motif and PPR protein]

### (Definition)

The PPR motif referred to in the present invention means a polypeptide constituted by 30 to 38 amino acids and having an amino acid sequence of an E value not larger than a predetermined value (desirably E-03) obtained for PF01535 in Pfam or PS51375 in Prosite as determined by amino acid sequence analysis with a protein domain search program on the Web, unless especially stated. The position numbers of amino acids constituting the PPR motif defined in the present invention are substantially synonymous with those of PF01535, and they correspond to those obtained by subtracting 2 from the numbers of the amino acid positions of PS51375 (for example, the position 1 referred to in the present invention corresponds to the position 3 of PS51375). Further, the term "ii" (-2)-th amino acid means the second amino acid from the end (C-terminus side) of the amino acids constituting the PPR motif, or the second amino acid towards the N-terminus side from the first amino acid of the following PPR motif, i.e., -2nd amino acid. When the following PPR motif is not definitely identified, the amino acid 2 amino acids before the first amino acid of the following helical structure is the amino acid of "ii". For Pfam, http://pfam.sanger.ac.uk/ can be referred to, and for Prosite, http://www.expasy.org/prosite/ can be referred to.

Although the conservativeness of the conserved amino acid sequence of the PPR motif is low at the amino acid level, two of the α-helixes as the secondary structure are well conserved. Although a typical PPR motif is constituted by 35 amino acids, the length thereof is as variable as is from 30 to 38 amino acids.

More specifically, the PPR motif referred to in the present invention consists of a polypeptide of a 30- to 38-amino acid length represented by the formula 1.

[Formula 1]

**(Helix A)-X-(Helix B)-L** (Formula 1)

In the formula:
Helix A is a moiety of 12-amino acid length capable of forming an α-helix structure, and is represented by the formula 2;
[Formula 2]

   **A₁-A₂-A₃-A₄-A₅-A₆-A₇-A₈-A₉-A₁₀-A₁₁-A₁₂** (Formula 2)

   wherein, in the formula 2, A₁ to A₁₂ independently represent an amino acid;
X does not exist, or is a moiety of 1- to 9-amino acid length;
Helix B is a moiety of 11- to 13-amino acid length capable of forming an α-helix structure; and
L is a moiety of 2- to 7-amino acid length represented by the formula 3;
[Formula 3]

   **Lᵥᵢᵢ-Lᵥᵢ-Lᵥ-Lᵢᵥ-LᵢᵢᵢLᵢᵢ-Lᵢ** (Formula 3)

   wherein, in the formula 3, the amino acids are numbered "i" (-1), "ii" (-2), and so on from the C-terminus side,
provided that Lᵢᵢᵢ to Lᵥᵢᵢ may not exist.

The term PPR protein used in the present invention refers to a PPR protein comprising one or more, preferably two or more, of the above-mentioned PPR motifs, unless especially indicated. The term protein used in this description refers to any substance consisting of a polypeptide (chain consisting of a plurality of amino acids bound via peptide bonds), unless especially indicated, and includes those consisting of a polypeptide of a comparatively low molecular weight. The term amino acid used in the present invention refers to a usual amino acid molecule, and also refers to an amino acid residue constituting a peptide chain. Which one is referred to shall be clear to those skilled in the art from the context.

In the present invention, the term specificity/specific used for the binding property of the PPR motif to a base in the target nucleic acid means that the binding activity to any one of the four bases is higher than the binding activities to the other bases, unless especially stated.

In the present invention, the term nucleic acid refers to RNA or DNA. Although the PPR protein may have specificity for bases in RNA or DNA, it does not bind to nucleic acid monomers.

In the PPR motif, combination of three of the 1st, 4th, and ii-th amino acids is important for specific binding to a base, and to which base the motif binds can be determined according to this combination (Patent document 1 and 2 mentioned above).

Specifically, with respect to the RNA-binding PPR motifs, the relationship between the combinations of three of the 1st, 4th, and ii-th amino acids and the bases to which they can bind is as follows (see Patent document 1 mentioned above).

(3-1) When the combination of the three amino acids of A₁, A₄, and Lᵢᵢ consists of valine, asparagine, and aspartic acid in this order, the PPR motif has such a selective RNA base-binding ability that the motif strongly binds to U, less strongly to C, and still less strongly to A or G.

(3-2) When the combination of the three amino acids of A₁, A₄, and Lᵢᵢ consists of valine, threonine, and asparagine in this order, the PPR motif has such a selective RNA base-binding ability that the motif strongly binds to A, less strongly to G, and still less strongly to C, but dose not bind to U.

(3-3) When the combination of the three amino acids of A₁, A₄, and Lᵢᵢ consists of valine, asparagine, and asparagine in this order, the PPR motif has such a selective RNA base-binding ability that the motif strongly binds to C, and less strongly to A or U, but does not bind to G.

(3-4) When the combination of the three amino acids of A₁, A₄, and Lᵢᵢ consists of glutamic acid, glycine, and aspartic acid in this order, the PPR motif has such a selective RNA base-binding ability that the motif strongly binds to G, but does not bind to A, U, and C.

(3-5) When the combination of the three amino acids of A₁, A₄, and Lᵢᵢ consists of isoleucine, asparagine, and asparagine in this order, the PPR motif has such a selective RNA base-binding ability that the motif strongly binds to C, less strongly to U, and still less strongly to A, but does not bind to G.

(3-6) When the combination of the three amino acids of A₁, A₄, and Lᵢᵢ consists of valine, threonine, and aspartic acid in this order, the PPR motif has such a selective RNA base-binding ability that the motif strongly binds to G, and less strongly to U, but does not bind to A and C.

(3-7) When the combination of the three amino acids of A₁, A₄, and Lᵢᵢ consists of lysine, threonine, and aspartic acid in this order, the PPR motif has such a selective RNA base-binding ability that the motif strongly binds to G, and less strongly to A, but does not bind to U and C.

(3-8) When the combination of the three amino acids of A₁, A₄, and Lᵢᵢ consists of phenylalanine, serine, and asparagine in this order, the PPR motif has such a selective RNA base-binding ability that the motif strongly binds to A, less strongly to C, and still less strongly to G and U.

(3-9) When the combination of the three amino acids of A₁, A₄, and Lᵢᵢ consists of valine, asparagine, and serine in this order, the PPR motif has such a selective RNA base-binding ability that the motif strongly binds to C, and less strongly to U, but does not bind to A and G.

(3-10) When the combination of the three amino acids of A₁, A₄, and Lᵢᵢ consists of phenylalanine, threonine, and asparagine in this order, the PPR motif has such a selective RNA base-binding ability that the motif strongly binds to A, but does not bind to G, U, and C.

(3-11) When the combination of the three amino acids of A₁, A₄, and Lᵢᵢ consists of isoleucine, asparagine, and aspartic acid in this order, the PPR motif has such a selective RNA base-binding ability that the motif strongly binds to U, and less strongly to A, but does not bind to G and C.

(3-12) When the combination of the three amino acids of A₁, A₄, and Lᵢᵢ consists of threonine, threonine, and asparagine in this order, the PPR motif has such a selective RNA base-binding ability that the motif strongly binds to A, but does not bind to G, U, and C.

(3-13) When the combination of the three amino acids of A₁, A₄, and Lᵢᵢ consists of isoleucine, methionine, and aspartic acid in this order, the PPR motif has such a selective RNA base-binding ability that the motif strongly binds to U, and less strongly to C, but does not bind to A and G.

(3-14) When the combination of the three amino acids of A₁, A₄, and Lᵢᵢ consists of phenylalanine, proline, and aspartic acid in this order, the PPR motif has such a selective RNA base-binding ability that the motif strongly binds to U, and less strongly to C, but does not bind to A and G.

(3-15) When the combination of the three amino acids of A₁, A₄, and Lᵢᵢ consists of tyrosine, proline, and aspartic acid in this order, the PPR motif has such a selective RNA base-binding ability that the motif strongly binds to U, but does not bind to A, G, and C.

(3-16) When the combination of the three amino acids of A₁, A₄, and Lᵢᵢ consists of leucine, threonine, and aspartic acid in this order, the PPR motif has such a selective RNA base-binding ability that the motif strongly binds to G, but does not bind to A, U, and C.

Specifically, with respect to the DNA-binding PPR motifs, the relationship between combinations of the three of the 1st, 4th, and ii-th amino acids and the bases to which they can bind is as follows (see Patent document 2 mentioned above).

(2-1) When the combination of the three amino acids of A₁, A₄, and Lᵢᵢ consists of an arbitrary amino acid, glycine, and aspartic acid in this order, the PPR motif selectively binds to G.

(2-2) When the combination of the three amino acids of A₁, A₄, and Lᵢᵢ consists of glutamic acid, glycine, and aspartic acid in this order, the PPR motif selectively binds to G.

(2-3) When the combination of the three amino acids of A₁, A₄, and Lᵢᵢ consists of an arbitrary amino acid, glycine, and asparagine in this order, the PPR motif selectively binds to A.

(2-4) When the combination of the three amino acids of A₁, A₄, and Lᵢᵢ consists of glutamic acid, glycine, and asparagine in this order, the PPR motif selectively binds to A.

(2-5) When the combination of the three amino acids of A₁, A₄, and Lᵢᵢ consists of an arbitrary amino acid, glycine, and serine in this order, the PPR motif selectively binds to A, and less selectively to C.

(2-6) When the combination of the three amino acids of A₁, A₄, and Lᵢᵢ consists of an arbitrary amino acid, isoleucine, and an arbitrary amino acid in this order, the PPR motif selectively binds to T and C.

(2-7) When the combination of the three amino acids of A₁, A₄, and Lᵢᵢ consists of an arbitrary amino acid, isoleucine, and asparagine in this order, the PPR motif selectively binds to T, and less selectively to C.

(2-8) When the combination of the three amino acids of A₁, A₄, and Lᵢᵢ consists of an arbitrary amino acid, leucine, and an arbitrary amino acid in this order, the PPR motif selectively binds to T and C.

(2-9) When the combination of the three amino acids of A₁, A₄, and Lᵢᵢ consists of an arbitrary amino acid, leucine, and aspartic acid in this order, the PPR motif selectively binds to C.

(2-10) When the combination of the three amino acids of A₁, A₄, and Lᵢᵢ consists of an arbitrary amino acid, leucine, and lysine in this order, the PPR motif selectively binds to T.

(2-11) When the combination of the three amino acids of A₁, A₄, and Lᵢᵢ consists of an arbitrary amino acid, methionine, and an arbitrary amino acid in this order, the PPR motif selectively binds to T.

(2-12) When the combination of the three amino acids of A₁, A₄, and Lᵢᵢ consists of an arbitrary amino acid, methionine, and aspartic acid in this order, the PPR motif selectively binds to T.

(2-13) When the combination of the three amino acids of A₁, A₄, and Lᵢᵢ consists of isoleucine, methionine, and aspartic acid in this order, the PPR motif selectively binds to T, and less selectively to C.

(2-14) When the combination of the three amino acids of A₁, A₄, and Lᵢᵢ consists of an arbitrary amino acid, asparagine, and an arbitrary amino acid in this order, the PPR motif selectively binds to C and T.

(2-15) When the combination of the three amino acids of A₁, A₄, and Lᵢᵢ consists of an arbitrary amino acid, asparagine, and aspartic acid in this order, the PPR motif selectively binds to T.

(2-16) When the combination of the three amino acids of A₁, A₄, and Lᵢᵢ consists of phenylalanine, asparagine, and aspartic acid in this order, the PPR motif selectively binds to T.

(2-17) When the combination of the three amino acids of A₁, A₄, and Lᵢᵢ consists of glycine, asparagine, and aspartic acid in this order, the PPR motif selectively binds to T.

(2-18) When the combination of the three amino acids of A₁, A₄, and Lᵢᵢ consists of isoleucine, asparagine, and aspartic acid in this order, the PPR motif selectively binds to T.

(2-19) When the combination of the three amino acids of A₁, A₄, and Lᵢᵢ consists of threonine, asparagine, and aspartic acid in this order, the PPR motif selectively binds to T.

(2-20) When the combination of the three amino acids of A₁, A₄, and Lᵢᵢ consists of valine, asparagine, and aspartic acid in this order, the PPR motif selectively binds to T, and less selectively to C.

(2-21) When the combination of the three amino acids of A₁, A₄, and Lᵢᵢ consists of tyrosine, asparagine, and aspartic acid in this order, the PPR motif selectively binds to T, and less selectively to C.

(2-22) When the combination of the three amino acids of A₁, A₄, and Lᵢᵢ consists of an arbitrary amino acid, asparagine, and asparagine in this order, the PPR motif selectively binds to C.

(2-23) When the combination of the three amino acids of A₁, A₄, and Lᵢᵢ consists of isoleucine, asparagine, and asparagine in this order, the PPR motif selectively binds to C.

(2-24) When the combination of the three amino acids of A₁, A₄, and Lᵢᵢ consists of serine, asparagine, and asparagine in this order, the PPR motif selectively binds to C.

(2-25) When the combination of the three amino acids of A₁, A₄, and Lᵢᵢ consists of valine, asparagine, and asparagine in this order, the PPR motif selectively binds to C.

(2-26) When the combination of the three amino acids of A₁, A₄, and Lᵢᵢ consists of an arbitrary amino acid, asparagine, and serine in this order, the PPR motif selectively binds to C.

(2-27) When the combination of the three amino acids of A₁, A₄, and Lᵢᵢ consists of valine, asparagine, and serine in this order, the PPR motif selectively binds to C.

(2-28) When the combination of the three amino acids of A₁, A₄, and Lᵢᵢ consists of an arbitrary amino acid, asparagine, and threonine in this order, the PPR motif selectively binds to C.

(2-29) When the combination of the three amino acids of A₁, A₄, and Lᵢᵢ consists of valine, asparagine, and threonine in this order, the PPR motif selectively binds to C.

(2-30) When the combination of the three amino acids of A₁, A₄, and Lᵢᵢ consists of an arbitrary amino acid, asparagine, and tryptophan in this order, the PPR motif selectively binds to C, and less selectively to T.

(2-31) When the combination of the three amino acids of A₁, A₄, and Lᵢᵢ consists of isoleucine, asparagine, and tryptophan in this order, the PPR motif selectively binds to T, and less selectively to C.

(2-32) When the combination of the three amino acids of A₁, A₄, and Lᵢᵢ consists of an arbitrary amino acid, proline, and an arbitrary amino acid in this order, the PPR motif selectively binds to T.

(2-33) When the combination of the three amino acids of A₁, A₄, and Lᵢᵢ consists of an arbitrary amino acid, proline, and aspartic acid in this order, the PPR motif selectively binds to T.

(2-34) When the combination of the three amino acids of A₁, A₄, and Lᵢᵢ consists of phenylalanine, proline, and aspartic acid in this order, the PPR motif selectively binds to T.

(2-35) When the combination of the three amino acids of A₁, A₄, and Lᵢᵢ consists of tyrosine, proline, and aspartic acid in this order, the PPR motif selectively binds to T.

(2-36) When the combination of the three amino acids of A₁, A₄, and Lᵢᵢ consists of an arbitrary amino acid, serine, and an arbitrary amino acid in this order, the PPR motif selectively binds to A and G.

(2-37) When the combination of the three amino acids of A₁, A₄, and Lᵢᵢ consists of an arbitrary amino acid, serine, and asparagine in this order, the PPR motif selectively binds to A.

(2-38) When the combination of the three amino acids of A₁, A₄, and Lᵢᵢ consists of phenylalanine, serine, and asparagine in this order, the PPR motif selectively binds to A.

(2-39) When the combination of the three amino acids of A₁, A₄, and Lᵢᵢ consists of valine, serine, and asparagine in this order, the PPR motif selectively binds to A.

(2-40) When the combination of the three amino acids of A₁, A₄, and Lᵢᵢ consists of an arbitrary amino acid, threonine, and an arbitrary amino acid in this order, the PPR motif selectively binds to A and G.

(2-41) When the combination of the three amino acids of A₁, A₄, and Lᵢᵢ consists of an arbitrary amino acid, threonine, and aspartic acid in this order, the PPR motif selectively binds to G.

(2-42) When the combination of the three amino acids of A₁, A₄, and Lᵢᵢ consists of valine, threonine, and aspartic acid in this order, the PPR motif selectively binds to G.

(2-43) When the combination of the three amino acids of A₁, A₄, and Lᵢᵢ consists of an arbitrary amino acid, threonine, and asparagine in this order, the PPR motif selectively binds to A.

(2-44) When the combination of the three amino acids of A₁, A₄, and Lᵢᵢ consists of phenylalanine, threonine, and asparagine in this order, the PPR motif selectively binds to A.

(2-45) When the combination of the three amino acids of A₁, A₄, and Lᵢᵢ consists of isoleucine, threonine, and asparagine in this order, the PPR motif selectively binds to A.

(2-46) When the combination of the three amino acids of A₁, A₄, and Lᵢᵢ consists of valine, threonine, and asparagine in this order, the PPR motif selectively binds to A.

(2-47) When the combination of the three amino acids of A₁, A₄, and Lᵢᵢ consists of an arbitrary amino acid, valine, and an arbitrary amino acid in this order, the PPR motif binds to A, C, and T, but does not bind to G.

(2-48) When the combination of the three amino acids of A₁, A₄, and Lᵢᵢ consists of isoleucine, valine, and aspartic acid in this order, the PPR motif selectively binds to C, and less selectively to A.

(2-49) When the combination of the three amino acids of A₁, A₄, and Lᵢᵢ consists of an arbitrary amino acid, valine, and glycine in this order, the PPR motif selectively binds to C.

(2-50) When the combination of the three amino acids of A₁, A₄, and Lᵢᵢ consists of an arbitrary amino acid, valine, and threonine in this order, the PPR motif selectively binds to T.

### (Novel PPR motifs)

The present invention provides novel PPR motifs. The novel PPR motifs having an adenine-binding property provided by the present invention are (A-1), (A-2), and (A-3) mentioned below:
(A-1) a PPR motif consisting of the sequence of SEQ ID NO: 9, or a PPR motif consisting of the sequence of SEQ ID NO: 9 having a substitution selected from the group consisting of substitution of the amino acid at position 10 with tyrosine, substitution of the amino acid at position 15 with lysine, substitution of the amino acid at position 16 with leucine, substitution of the amino acid at position 17 with glutamic acid, substitution of the amino acid at position 18 with aspartic acid, and substitution of the amino acid at position 28 with glutamic acid;
(A-2) a PPR motif consisting of the sequence of SEQ ID NO: 9 having a substitution, deletion, or addition of 1 to 20 amino acids other than the amino acids at positions 1, 2, 3, 4, 6, 7, 9, 11, 12, 14, 19, 26, 30, 33, and 34, and having an adenine-binding property; and
(A-3) a PPR motif having a sequence identity of at least 42% to the sequence of SEQ ID NO: 9, provided that the amino acids at positions 1, 2, 3, 4, 6, 7, 9, 11, 12, 14, 19, 26, 30, 33, and 34 are identical, and having an adenine-binding property;

The substitution in the motif of (A-1) may consist of one, two or more, or all of the substitutions mentioned above.

In the motif of (A-2), 1 to 20 amino acids other than the amino acids at positions 1, 2, 3, 4, 6, 7, 9, 11, 12, 14, 19, 26, 30, 33, and 34, which are amino acids in the sequence of SEQ ID NO: 9 that may be substituted or the like are:
preferably, 1 to 11 amino acids other than the amino acids at positions 1, 2, 3, 4, 6, 7, 9, 11, 12, 14, 19, 26, 30, 33, and 34, and other than the amino acids at positions 5, 8, 13, 21, 22, 23, 25, 29, and 35,
more preferably, 1 to 7 amino acids other than the amino acids at positions 1, 2, 3, 4, 6, 7, 9,11, 12, 14, 19, 26, 30, 33, and 34, other than the amino acids at positions 5, 8, 13, 21, 22, 23, 25, 29, and 35, and other than the amino acids at positions 20, 24, 31, and 32,
further preferably, any of the amino acids at positions 10, 15, 16, 17, 18, and 28.

The characteristic of the motif (A-3) of having a sequence identity of at least 42% to the sequence of SEQ ID NO: 9, provided that the amino acids at positions 1, 2, 3, 4, 6, 7, 9, 11, 12, 14, 19, 26, 30, 33, and 34 are identical is:
preferably, to have a sequence identity of at least 71% to the sequence of SEQ ID NO: 9, provided that the amino acids at positions 1, 2, 3, 4, 6, 7, 9, 11, 12, 14, 19, 26, 30, 33, and 34, and the amino acids at positions 5, 8, 13, 21, 22, 23, 25, 29, and 35 are identical,
more preferably, to have a sequence identity of at least 80% to the sequence of SEQ ID NO: 9, provided that the amino acids at positions 1, 2, 3, 4, 6, 7, 9, 11, 12, 14, 19, 26, 30, 33, and 34, the amino acids at positions 5, 8, 13, 21, 22, 23, 25, 29, and 35, and the amino acids at positions 20, 24, 31 and 32 are identical,
still more preferably, to have a sequence identity of at least 82% to the sequence of SEQ ID NO: 9, provided that the amino acid not identical is any of the amino acids at positions 10, 15, 16, 17, 18, and 28.

Novel PPR motifs and having a cytosine-binding property provided by the present invention are (C-1), (C-2), and (C-3) mentioned below:
(C-1) a PPR motif consisting of the sequence of SEQ ID NO: 10, or a PPR motif consisting of the sequence of SEQ ID NO: 10 having a substitution of amino acid selected from the group consisting of substitution of the amino acid at position 2 with serine, substitution of the amino acid at position 5 with isoleucine, substitution of the amino acid at position 7 with leucine, substitution of the amino acid at position 8 with lysine, substitution of the amino acid at position 10 with phenylalanine or tyrosine, substitution of the amino acid at position 15 with arginine, substitution of the amino acid at position 22 with valine, substitution of the amino acid at position 24 with arginine, substitution of the amino acid at position 27 with leucine, and substitution of the amino acid at position 29 with arginine;
(C-2) a PPR motif consisting of the sequence of SEQ ID NO: 10 having a substitution, deletion, or addition of 1 to 25 amino acids other than the amino acids at positions 1, 3, 4, 14, 18, 19, 26, 30, 33, and 34, and having a cytosine-binding property; and
(C-3) a PPR motif having a sequence identity of at least 25% to the sequence of SEQ ID NO: 10, provided that the amino acids at positions 1, 3, 4, 14, 18, 19, 26, 30, 33, and 34 are identical, and having a cytosine-binding property;

The substitution in the motif (C-1) may consist of one, two or more, or all of the substitutions mentioned.

In the motif (C-2), 1 to 25 of the amino acids other than the amino acids at positions 1, 3, 4, 14, 18, 19, 26, 30, 33, and 34, which are amino acids that may be substituted or the like, in the sequence of SEQ ID NO: 10 are
preferably, 1 to 14 amino acids other than the amino acids at positions 1, 3, 4, 14, 18, 19, 26, 30, 33, and 34, and other than the amino acids at positions 6, 9, 11, 12, 17, 20, 21, 23, 25, 28, and 35,
more preferably, 1 to 10 amino acids other than the amino acids at positions 1, 3, 4, 14, 18, 19, 26, 30, 33, and 34, other than the amino acids at positions 6, 9, 11, 12, 17, 20, 21, 23, 25, 28, and 35, and other than the amino acids at positions 13, 16, 31, and 32,
still more preferably, any of the amino acids at positions 2, 5, 7, 8, 10, 15, 22, 24, 27, and 29.

The characteristic of the motif (C-3) of having a sequence identity of at least 25% to the sequence of SEQ ID NO: 10, provided that the amino acids at positions 1, 3, 4, 14, 18, 19, 26, 30, 33, and 34 are identical is:
preferably, to have a sequence identity of at least 60% to the sequence of SEQ ID NO: 10, provided that the amino acids at positions 1, 3, 4, 14, 18, 19, 26, 30, 33, and 34 and the amino acids at 6, 9, 11, 12, 17, 20, 21, 23, 25, 28, and 35 are identical,
more preferably, to have a sequence identity of at least 71% to the sequence of SEQ ID NO: 10, provided that the amino acids at positions 1, 3, 4, 14, 18, 19, 26, 30, 33, and 34, the amino acids at positions 6, 9, 11, 12, 17, 20, 21, 23, 25, 28, and 35, and the amino acids at positions 13, 16, 31 and 32 are identical,
still more preferably, to have a sequence identity of at least 71% to the sequence of SEQ ID NO: 10, provided that the amino acid that is not identical are any of the amino acids at positions 2, 5, 7, 8, 10, 15, 22, 24, 27, and 29.

Novel PPR motifs having a guanine-binding property provided by the present invention and are (G-1),(G-2), and (G-3) mentioned below:
(G-1) a PPR motif consisting of the sequence of SEQ ID NO: 11, or a PPR motif consisting of the sequence of SEQ ID NO: 11 having a substitution selected from the group consisting of substitution of the amino acid at position 10 with phenylalanine, substitution of the amino acid at position 15 with aspartic acid, substitution of the amino acid at position 27 with valine, substitution of the amino acid at position 28 with serine, and substitution of the amino acid at position 35 with isoleucine;
(G-2) a PPR motif consisting of the sequence of SEQ ID NO: 11 having a substitution, deletion, or addition of 1 to 21 amino acids other than the amino acids at positions 1, 2, 3, 4, 6, 7, 9, 14, 18, 19, 26, 30, 33, and 34, and having a guanine-binding property; and
(G-3) a PPR motif having a sequence identity of at least 40% to the sequence of SEQ ID NO: 11, provided that the amino acids at positions 1, 2, 3, 4, 6, 7, 9, 14, 18, 19, 26, 30, 33, and 34 are identical, and having a guanine-binding property.

The substitution in the motif of (G-1) may consist of one, two or more, or all of the substitutions mentioned above.

In the motif of (G-2), 1 to 21 amino acids other than the amino acids at positions 1, 2, 3, 4, 6, 7, 9, 14, 18, 19, 26, 30, 33, and 34, which are amino acids that may be substituted or the like, in the sequence of SEQ ID NO: 11 are:
preferably, 1 to 12 amino acids other than the amino acids at positions 1, 2, 3, 4, 6, 7, 9, 14, 18, 19, 26, 30, 33, and 34, and other than the amino acids at positions 5, 11, 12, 17, 20, 21, 22, 23, and 25,
more preferably, 1 to 5 amino acids other than the amino acids at positions 1, 2, 3, 4, 6, 7, 9, 14, 18, 19, 26, 30, 33, and 34, other than the amino acids at positions 5, 11, 12, 17, 20, 21, 22, 23, and 25, and other than the amino acids at positions 8, 13, 16, 24, 29, 31, and 32,
still more preferably, any of the amino acids at positions 10, 15, 27, 28, and 35.

The characteristic of the motif (G-3) of having a sequence identity of at least 40% to the sequence of SEQ ID NO: 11, provided that the amino acids at positions 1, 2, 3, 4, 6, 7, 9, 14, 18, 19, 26, 30, 33, and 34 are identical is:
preferably, to have a sequence identity of at least 65% to the sequence of SEQ ID NO: 11, provided that the amino acids at positions 1, 2, 3, 4, 6, 7, 9, 14, 18, 19, 26, 30, 33, and 34, and the amino acids at positions 5, 11, 12, 17, 20, 21, 22, 23, and 25 are identical;
more preferably, to have a sequence identity of at least 85% to the sequence of SEQ ID NO: 11, provided that the amino acids at positions 1, 2, 3, 4, 6, 7, 9, 14, 18, 19, 26, 30, 33, and 34, the amino acids at positions 5, 11, 12, 17, 20, 21, 22, 23, and 25, and the amino acids at positions 8, 13, 16, 24, 29, 31, and 32 are identical,
still more preferably, to have a sequence identity of at least 85% to the sequence of SEQ ID NO: 11, provided that the amino acid not identical is any of the amino acids at positions 10, 15, 27, 28, and 35.

Novel PPR motifs having a uracil-binding property provided by the present invention are the motifs of (U-1), (U-2), and (U-3) mentioned below:
(U-1) a PPR motif consisting of the sequence of SEQ ID NO: 12, or a PPR motif consisting of the sequence of SEQ ID NO: 12 having a substitution selected from the group consisting of substitution of the amino acid at position 10 with phenylalanine, substitution of the amino acid at position 13 with serine, substitution of the amino acid at position 15 with lysine, substitution of the amino acid at position 17 with glutamic acid, substitution of the amino acid at position 20 with leucine, substitution of the amino acid at position 21 with lysine, substitution of the amino acid at position 23 with phenylalanine, substitution of the amino acid at position 24 with aspartic acid, substitution of the amino acid at position 27 with lysine, substitution of the amino acid at position 28 with lysine, substitution of the amino acid at position 29 with arginine, and substitution of the amino acid at position 31 with leucine;
(U-2) a PPR motif consisting of the sequence of SEQ ID NO: 12 having a substitution, deletion, or addition of 1 to 22 amino acids other than the amino acids at positions 1, 2, 3, 4, 6, 11, 12, 14, 19, 26, 30, 33, and 34, and having a uracil-binding property; and
(U-3) a PPR motif having a sequence identity of at least 37% to the sequence of SEQ ID NO: 12, provided that the amino acids at positions 1, 2, 3, 4, 6, 11, 12, 14, 19, 26, 30, 33, and 34 are identical, and having a uracil-binding property.

The substitution in the motif (U-1) may consist of one, two or more, or all of the substitutions mentioned above.

In the motif of (U-2), 1 to 22 amino acids other than the amino acids at positions 1, 2, 3, 4, 6, 11, 12, 14, 19, 26, 30, 33, and 34, which are amino acids that may be substituted or the like, in the sequence of SEQ ID NO: 12 are
preferably, 1 to 14 amino acids other than the amino acids at positions 1, 2, 3, 4, 6, 11, 12, 14, 19, 26, 30, 33, and 34, and other than the amino acids at positions 5, 7, 9, 16, 18, 22, 25, and 35,
more preferably, 1 to 12 amino acids other than the amino acids at positions 1, 2, 3, 4, 6, 11, 12, 14, 19, 26, 30, 33, and 34, other than the amino acids at positions 5, 7, 9, 16, 18, 22, 25, and 35, and other than the amino acids at positions 8 and 32,
still more preferably, any of the amino acids at positions 10, 13, 15, 17, 20, 21, 23, 24, 27, 28, 29, and 31.

The characteristic of the motif (U-3) of having a sequence identity of at least 37% to the sequence of SEQ ID NO: 12, provided that the amino acids at positions 1, 2, 3, 4, 6, 11, 12, 14, 19, 26, 30, 33, and 34 are identical, is:
preferably, to have a sequence identity of at least 60% to the sequence of SEQ ID NO: 12, provided that the amino acids at positions 1, 2, 3, 4, 6, 11, 12, 14, 19, 26, 30, 33, and 34, and the amino acids at positions 5, 7, 9, 16, 18, 22, 25, and 35 are identical;
more preferably, to have a sequence identity of at least 65% to the sequence of SEQ ID NO: 12, provided that the amino acids at positions 1, 2, 3, 4, 6, 11, 12, 14, 19, 26, 30, 33, and 34, the amino acids at positions 5, 7, 9, 16, 18, 22, 25, and 35, and the amino acids at positions 8 and 32 are identical,
still more preferably, to have a sequence identity of at least 65% to the sequence of SEQ ID NO: 12, provided that the amino acid not identical is any of the amino acids at positions 10, 13, 15, 17, 20, 21, 23, 24, 27, 28, 29, and 31.

The PPR motifs v2_A (SEQ ID NO: 9), v2_C (SEQ ID NO: 10), v2_G (SEQ ID NO: 11), and v2_U (SEQ ID NO: 12), which were created by the inventors of the present invention, are disclosed for the first time by this application, and do not exist in nature. As for homologues thereof (the embodiments mentioned above as (A-1), (A-2), (A-3), (C-1), (C-2), (C-3), (G-1), (G-2), (G-3), (U-1), (U-2), and (U-3) and preferred embodiments thereof that comprises a sequence other than those of SEQ ID NOS: 9 to 12), it is considered that combinations of at least any two or more, e.g., 3, 4, 5, 6, 7, 8, 9, 10, 11, 12, 13, or 14, of the homologues do not exist in the nature (irrespective of whether or not the individual homologues are disclosed for the first time by this application, and whether or not they exist in the nature). In the present invention, the number meant by the term "any" may be an arbitrary number.

### (Explanation of sequences of novel PPR motifs)

Figs. 1 to 4 summarize types and occurring numbers of amino acids at every position in the *Arabidopsis thaliana* PPR motif sequences, for which there were collected the PPR motifs in which the combination of amino acids locating at positions 1, 4, and ii is VTN as adenine-recognizing PPR motifs, those in which the same is VSN as the cytosine-recognizing PPR motifs, those in which the same is VTD as the guanine-recognizing PPR motifs, and those in which the same is VND as the uracil-recognizing uracil PPR motifs. The amino acids at all the positions in the sequences of the novel PPR motif sequences v2_A (SEQ ID NO: 9), v2_C (SEQ ID NO: 10), v2_G (SEQ ID NO: 11), and v2_U (SEQ ID NO: 12) are those of high occurrence frequency. In Fig. 6A, along with these novel sequences, v1_A (SEQ ID NO: 13), v1_C (SEQ ID NO: 14), v1_G (SEQ ID NO: 15), and v1_U (SEQ ID NO: 16) are also shown, which sequences have the dPPR motif in which the combination of the amino acids at positions 1, 4, and ii is the same as that of v2.

Fig. 6A also shows the amino acid sequence of the v3.1 motif. v3.1 is the same as v2 except that a D15K mutation is introduced into the adenine-recognizing motif of v2 (SEQ ID NO: 401), and thus the other parts of them are identical. Use of v3.1 in PPR proteins may provide those showing improved binding power compared with v2.

Further, Tables 1 to 4 mentioned below summarize magnitudes of deviations of occurrence frequency of the amino acids in the sequences of SEQ ID NOS: 9 to 12 from random occurrence (e.g., if 100 PPR motifs are collected, and it is supposed that the amino acids randomly occur at a certain position as for the occurrence frequency of the amino acids, each of the 20 types of amino acids should appear 5 times at that position). If the occurrence frequency of amino acid at a certain position is deviated from random occurrence and high, it is considered that the amino acid at that position is evolutionarily converged, and highly related to the function. Even if an amino acid is substituted with another type of amino acid of which occurrence frequency is deviated from random occurrence, and of which occurrence frequency is high, the function of the PPR motif can be maintained, so long as the amino acid highly relates to the function.

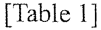

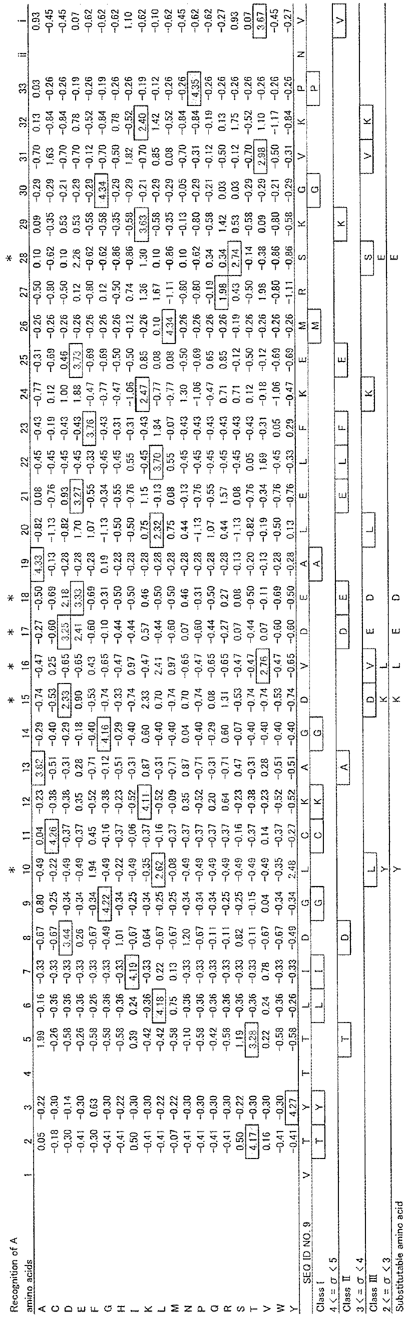

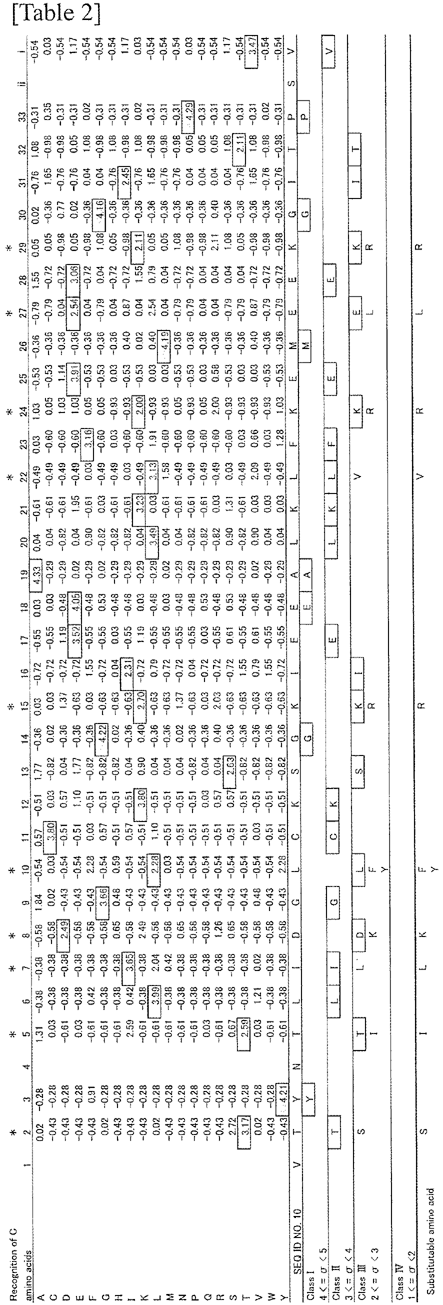

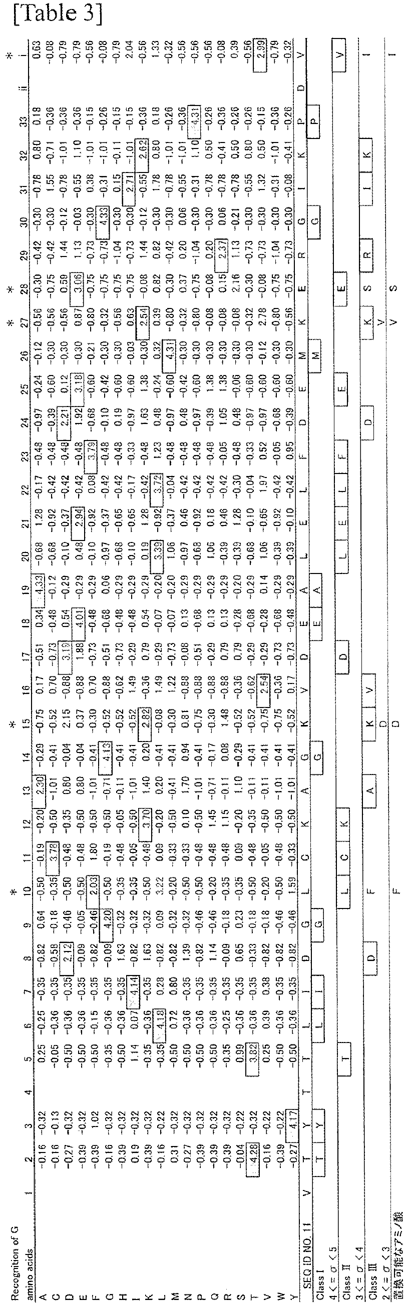

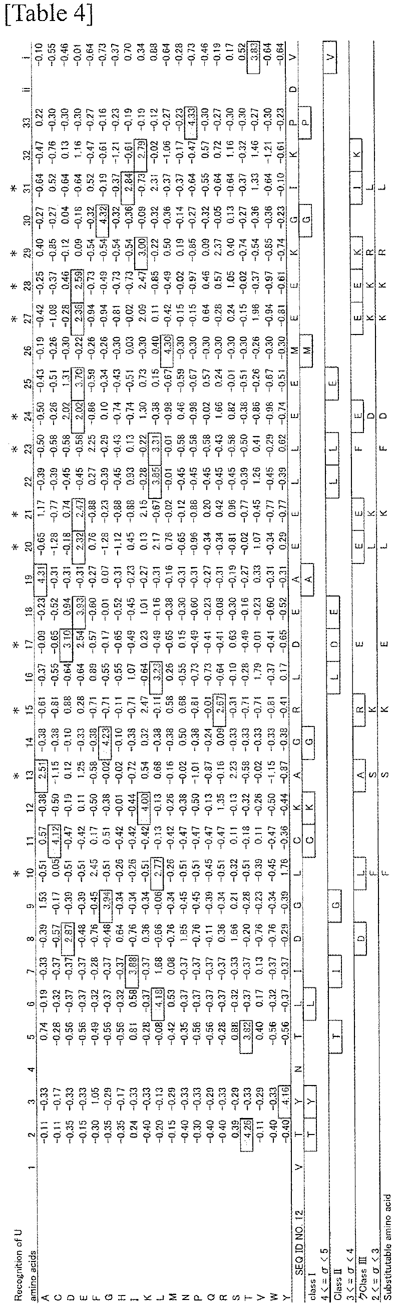

### (Novel PPR protein)

The present invention provides novel PPR proteins containing a novel PPR motif.

The novel PPR proteins provided by the present invention are those mentioned below.

A protein comprising n of PPR motifs and capable of binding to a target RNA consisting of a sequence of n bases in length, wherein:
the PPR motif for adenine in the base sequence is the PPR motif of (A-1), (A-2), or (A-3) mentioned above;
the PPR motif for cytosine in the base sequence is the PPR motif of (C-1), (C-2), or (c-3) mentioned above;
the PPR motif for guanine in the base sequence is the PPR motif of (G-1), (G-2), or (G-3) mentioned above; and
the PPR motif for uracil in the base sequence is the PPR motif of (U-1), (U-2), or (U-3) mentioned above.

As for preferred examples of the PPR motifs contained in the PPR proteins, the descriptions concerning the PPR motifs for (A-1), (A-2), (A-3), (C-1), (C-2), (C-3), (G-1), (G-2), (G-3), (U-1), (U-2), or (U-3) mentioned above can be applied as they are.

In the PPR protein of the present invention, n (representing an integer of 1 or larger) is not particularly limited, but can be 10 or larger, preferably 12 or larger, more preferably 15 or larger, still more preferably 18 or larger. An increased number of the motifs allows preparation of a PPR protein showing a high binding strength to larger number of kinds of targets.

While preparations of artificial PPR proteins comprising of 7 to 14 motifs have so far been reported as shown in the tables mentioned below, it has been considered that construction of a gene for a PPR protein containing a larger number of PPR motifs, which should inevitably result in larger number of repeats in nucleotide sequence of the gene sequence encoding such a protein. In general, it may be difficult to prepare genes containing repeat sequences, because, for example, the repeat moieties are recombined during the cloning process (Trinh, T. et al., An Escherichia coli strain for the stable propagation of retroviral clones and direct repeat sequences, Focus, 16, 78-80 (1994)).

In the table, the Kd values are the lowest values among those given in the literature.

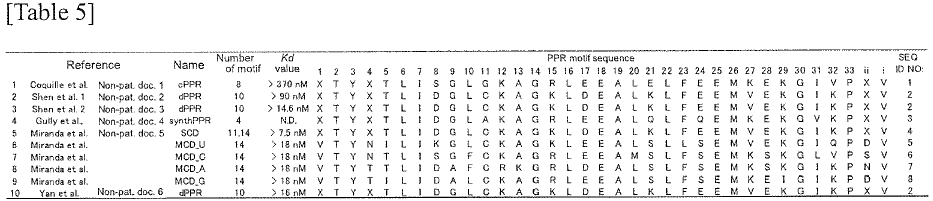

When constructing a gene for a PPR protein having 15 or more PPR motifs, a gene in which number of repeat is reduced can be constructed by appropriately changing nucleotide sequences encoding amino acids other than the amino acids at positions of 1, 4, and ii responsible to the binding (when the Golden Gate method described below is used, the 5th to 33rd 29 of amino acids other than the common both end regions) among the motifs using codon degeneracy. Magnitude of the change can be appropriately determined by those skilled in the art, and for example, 4.5% or more (at more than 4 positions in 87 bases), 15% or more, or 30% or more (at more than 26 positions in 87 bases) of the bases can be changed.

For example, examples of nucleotide sequences encoding a motif obtained by utilizing codon degeneracy from the existing sequences encoding v1 to v4 motifs (SEQ ID NOS: 13 to 16) include those sequences shown in the table mentioned below.

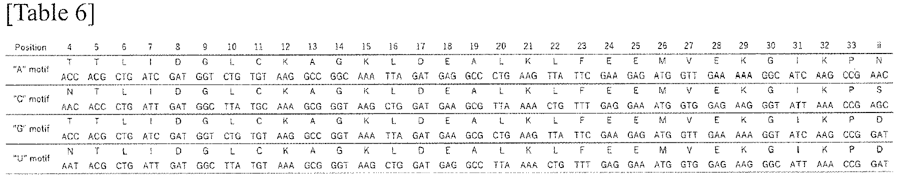

The term "preparation" can be rephrased as "production" or "manufacturing". In addition, the term "construction" is sometimes used to refer to preparation of a gene or the like by combining parts, and "construction" can also be rephrased as "production" or "manufacturing".

### (Nucleic acids encoding PPR motif and PPR protein)

The present invention provides nucleic acids encoding novel PPR motifs and novel PPR proteins containing the motifs. There are several variations in the nucleic acid sequences encoding the novel PPR motifs due to codon degeneracy.

Preferred examples of nucleotide sequences encoding the amino acid sequences of the novel PPR motifs of the present invention, v2_A (SEQ ID NO: 9), v2_C (SEQ ID NO: 10), v2_G (SEQ ID NO: 11), and v2_U (SEQ ID NO: 12), are shown in the table mentioned below.

**[Table 7-1]**

| Name | Nucleotide sequence | SEQ ID NO: |
|---|---|---|
| v2_A1_1 | GTCACATACACCACACTGATCGACGGACTGTGTAAAGCCGGCGACGTGGACGAAG CCCTCGAGCTGTTCAAAGAGATGCGGAGCAAGGGCGTGAAGCCCAACGTG | 358 |
| v2_C1_1 | GTCACATACAACACCCTGATCGACGGCCTGTGCAAGGCCGGCAGACTGGATGAGG CCGAGGAGCTGCTGGAGGAGATGGAGGAGAAGGGCATCAAGCCCGACGTG | 359 |
| v2_Gl_1 | GTCACATACACCACCCTGATCGACGGCCTGTGCAAGGCCGGCAAGGTGGATGAGG CCCTGGAGCTGTTCGACGAGATGAAGGAGAGGGGCATCAAGCCCGACGTG | 360 |
| v2_U1_1 | GTCACATACAACACCCTGATCGACGGCCTGTGCAAGAGCGGCAAGATCGAGGAGG CCCTGAAGCTGTTCAAGGAGATGGAGGAGAAGGGCATCACCCCCAGCGTG | 361 |
| v2_G2_1 | GTCACATACACCACCCTGATCGACGGCCTGTGCAAGGCCGGCAAAGTGGACGAGG CCCTGGAGCTGTTCGACGAGATGAAGGAGAGGGGCATCAAGCCCGACGTG | 362 |
| v2_A1_2 | GTGACATACACCACACTGATCGACGGACTGTGTAAAGCCGGCGACGTGGACGAAG CCCTCGAGCTGTTCAAAGAGATGCGGAGCAAGGGCGTGAAGCCCAACGTG | 363 |
| v2_C1_2 | GTGACATACAACACCCTGATCGACGGCCTGTGCAAGGCCGGCAGACTGGATGAGG CCGAGGAGCTGCTGGAGGAGATGGAGGAGAAGGGCATCAAGCCCGACGTG | 364 |
| v2_G1_2 | GTGACATACACCACCCTGATCGACGGCCTGTGCAAGGCCGGCAAGGTGGATGAGG CCCTGGAGCTGTTCGACGAGATGAAGGAGAGGGGCATCAAGCCCGACGTG | 365 |
| v2_U1_2 | GTGACATACAACACCCTGATCGACGGCCTGTGCAAGAGCGGCAAGATCGAGGAGG CCCTGAAGCTGTTCAAGGAGATGGAGGAGAAGGGCATCACCCCCAGCGTG | 366 |
| v2_G2_2 | GTGACATACACCACCCTGATCGACGGCCTGTGCAAGGCCGGCAAAGTGGACGAGG CCCTGGAGCTGTTCGACGAGATGAAGGAGAGGGGCATCAAGCCCGACGTG | 367 |
| v2_A1_3 | GTTACATACACCACACTGATCGACGGACTGTGTAAAGCCGGCGACGTGGACGAAG CCCTCGAGCTGTTCAAAGAGATGCGGAGCAAGGGCGTGAAGCCCAACGTG | 368 |
| v2_C1_3 | GTTACATACAACACCCTGATCGACGGCCTGTGCAAGGCCGGCAGACTGGATGAGG CCGAGGAGCTGCTGGAGGAGATGGAGGAGAAGGGCATCAAGCCCGACGTG | 369 |
| v2_G1_3 | GTTACATACACCACCCTGATCGACGGCCTGTGCAAGGCCGGCAAGGTGGATGAGG CCCTGGAGCTGTTCGACGAGATGAAGGAGAGGGGCATCAAGCCCGACGTG | 370 |
| v2_U1_3 | GTTACATACAACACCCTGATCGACGGCCTGTGCAAGAGCGGCAAGATCGAGGAGG CCCTGAAGCTGTTCAAGGAGATGGAGGAGAAGGGCATCACCCCCAGCGTG | 371 |
| v2_G2_3 | GTTACATACACCACCCTGATCGACGGCCTGTGCAAGGCCGGCAAAGTGGACGAGG CCCTGGAGCTGTTCGACGAGATGAAGGAGAGGGGCATCAAGCCCGACGTG | 372 |

The nucleotide sequences encoding the amino acid sequences of the PPR motifs v1_A (SEQ ID NO: 13), v1_C (SEQ ID NO: 14), v1_G (SEQ ID NO: 15), and v1_U (SEQ ID NO: 16), which correspond to the dPPR motif having the same combination of the amino acids at positions 1, 4, and ii as that of v2, are shown in the table mentioned below.

**[Table 7-2]**

| Name | Nucleotide sequence | SEQ ID NO: |
|---|---|---|
| v1_A1_1 | GTCACATACACCACGCTGATCGATGGTCTGTGTAAGGCCGGCAAATTAGATGAGG CCCTGAAGTTATTCGAAGAGATGGTTGAAAAAGGCATCAAGCCGAACGTG | 373 |
| v1_C1_1 | GTCACATACAACACCCTGATTGATGGCTTATGCAAAGCGGGTAAGCTGGATGAAG CGTTAAAACTGTTTGAGGAAATGGTGGAGAAGGGTATTAAACCGAGCGTG | 374 |
| v1_G1_1 | GTCACATACACCACGCTGATCGATGGTCTGTGTAAGGCCGGTAAATTAGATGAAG CGCTGAAGTTATTCGAAGAGATGGTTGAAAAAGGTATCAAGCCGGATGTG | 375 |
| V1_U1_1 | GTCACATACAATACGCTGATTGATGGCTTATGTAAAGCGGGTAAGCTGGATGAGG CCTTAAAACTGTTTGAGGAAATGGTGGAGAAGGGCATTAAACCGGATGTG | 376 |
| vl_G2_1 | GTCACATACACCACGCTGATCGATGGTCTGTGTAAGGCCGGTAAATTAGATGAAG CGCTGAAGTTATTCGAAGAGATGGTTGAAAAAGGTATCAAGCCGGACGTG | 377 |
| v1_U2_1 | GTCACATACAATACGCTGATTGATGGCTTATGTAAAGCGGGTAAGCTGGATGAGG CCTTAAAACTGTTTGAGGAAATGGTGGAGAAGGGCATTAAACCGGACGTG | 378 |
| v1_A1_2 | GTGACATACACCACGCTGATCGATGGTCTGTGTAAGGCCGGCAAATTAGATGAGG CCCTGAAGTTATTCGAAGAGATGGTTGAAAAAGGCATCAAGCCGAACGTG | 379 |
| v1_C1_2 | GTGACATACAACACCCTGATTGATGGCTTATGCAAAGCGGGTAAGCTGGATGAAG CGTTAAAACTGTTTGAGGAAATGGTGGAGAAGGGTATTAAACCGAGCGTG | 380 |
| v1_G1_2 | GTGACATACACCACGCTGATCGATGGTCTGTGTAAGGCCGGTAAATTAGATGAAG CGCTGAAGTTATTCGAAGAGATGGTTGAAAAAGGTATCAAGCCGGATGTG | 381 |
| v1_U1_2 | GTGACATACAATACGCTGATTGATGGCTTATGTAAAGCGGGTAAGCTGGATGAGG CCTTAAAACTGTTTGAGGAAATGGTGGAGAAGGGCATTAAACCGGATGTG | 382 |
| v1_G2_2 | GTGACATACACCACGCTGATCGATGGTCTGTGTAAGGCCGGTAAATTAGATGAAG CGCTGAAGTTATTCGAAGAGATGGTTGAAAAAGGTATCAAGCCGGACGTG | 383 |
| v1_U2_2 | GTGACATACAATACGCTGATTGATGGCTTATGTAAAGCGGGTAAGCTGGATGAGG CCTTAAAACTGTTTGAGGAAATGGTGGAGAAGGGCATTAAACCGGACGTG | 384 |
| v1_A1_3 | GTTACATACACCACGCTGATCGATGGTCTGTGTAAGGCCGGCAAATTAGATGAGG CCCTGAAGTTATTCGAAGAGATGGTTGAAAAAGGCATCAAGCCGAACGTG | 385 |
| v1_C1_3 | GTTACATACAACACCCTGATTGATGGCTTATGCAAAGCGGGTAAGCTGGATGAAG CGTTAAAACTGTTTGAGGAAATGGTGGAGAAGGGTATTAAACCGAGCGTG | 386 |
| v1_81_3 | GTTACATACACCACGCTGATCGATGGTCTGTGTAAGGCCGGTAAATTAGATGAAG CGCTGAAGTTATTCGAAGAGATGGTTGAAAAAGGTATCAAGCCGGATGTG | 387 |
| v1_U1_3 | GTTACATACAATACGCTGATTGATGGCTTATGTAAAGCGGGTAAGCTGGATGAGG CCTTAAAACTGTTTGAGGAAATGGTGGAGAAGGGCATTAAACCGGATGTG | 388 |
| v1_G2_3 | GTTACATACACCACGCTGATCGATGGTCTGTGTAAGGCCGGTAAATTAGATGAAG CGCTGAAGTTATTCGAAGAGATGGTTGAAAAAGGTATCAAGCCGGACGTG | 389 |
| v1_U2_3 | GTTACATACAATACGCTGATTGATGGCTTATGTAAAGCGGGTAAGCTGGATGAGG CCTTAAAACTGTTTGAGGAAATGGTGGAGAAGGGCATTAAACCGGACGTG | 390 |

The nucleotide sequence encoding the PPR protein can be constituted by any combination of the sequences mentioned above. The nucleotide sequence encoding the amino acids of the protein may be constituted by appropriately combining the nucleotide sequences encoding the amino acid sequences v2_A (SEQ ID NO: 9), v2_C (SEQ ID NO: 10), v2_G (SEQ ID NO: 11), and v2_U (SEQ ID NO: 12), and the nucleotide sequences encoding the amino acid sequences v1_A (SEQ ID NO: 13), v1_C (SEQ ID NO: 14), v1_G (SEQ ID NO: 15), and v1_U (SEQ ID NO: 16).

Preferred examples of the nucleotide sequences encoding the amino acid sequences v3.1_A (SEQ ID NO: 401), 1st_A (SEQ ID NO: 402), 1st_C (SEQ ID NO: 403), 1st_G (SEQ ID NO: 404), and 1st_U (SEQ ID NO: 405) of the novel PPR motifs of the present invention are shown in the table mentioned below.

**[Table 8]**

| Name | Nucleotide sequence | SEQ ID NO: |
|---|---|---|
| v3.1_A | GTGACCTACACCACACTGATCGACGGACTGTGCAAGGCCGGCAAAGTGGATGAGG CTCTGGAGCTGTTTAAGGAAATGAGAAGCAAGGGCGTCAAGCCCAACGTG | 406 |
| v3.2_A (1st_A) | GTCACATACACCACCAACATCGACCAGCTGTGCAAAGCCGGCAAGGTGGATGAAG CTCTGGAGCTGTTCAAGGAGATGAGAAGCAAGGGCGTGAAGCCCAACGTG | 407 |
| v3.2_C (1st_C) | GTCACATACAACACCAACATCGACCAGCTGTGCAAAAGCGGCAAGATCGAGGAGG CTCTGAAACTGTTCAAGGAGATGGAGGAGAAGGGCATCACCCCCAGCGTG | 408 |
| v3.2_G (1st_G) | GTCACATACACCACCAACATCGACCAGCTCTGCAAGGCCGGCAAGGTGGATGAGG CTCTGGAGCTGTTCGACGAGATGAAGGAGAGAGGCATCAAGCCCGACGTG | 409 |
| v3.2_U (1st_U) | GTCACATACAACACCAACATCGACCAGCTCTGCAAGGCCGGCAGACTGGACGAGG CCGAAGAGCTGCTGGAGGAGATGGAGGAGAAGGGCATCAAGCCCGACGTG | 410 |

The nucleotide sequence encoding the PPR protein can be constituted by any combination of the sequences mentioned above. There may be chosen any one selected from v3.2_X mentioned above as the nucleotide sequence encoding the first PPR motif from the N-terminus, then for the nucleotide sequences encoding the following PPR motifs, v3.1_A mentioned above as the nucleotide sequence encoding the PPR motif for adenine, and those selected from the v2 series mentioned above as the nucleotide sequences encoding the PPR motifs for cytosine, guanine, and uracil, and they can be appropriately combined.

### (Improvement of aggregation property)

The inventors of the present invention found that the amino acid at position 6 of the PPR motif is extremely frequently hydrophobic amino acid (especially leucine) and the amino acid at position 9 is extremely frequently a non-hydrophilic amino acid (especially glycine) on the basis of the amino acid information of existing naturally occurring PPR motifs. On the basis of structures of the PPR proteins for which crystal structures have already been obtained (Non-patent document 6: Coquille et al., 2014 Nat. Commun., PDB ID: 4PJQ, 4WN4, 4WSL, 4PJR, Non-patent document 7: Shen et al., 2015 Nat. Commun., PDB ID: 519D, 5I9F, 5I9G, 5I9H), they imagined that since those 6th and 9th amino acids in the first motif (N-terminus side) are exposed to the outside, the proteins show aggregation property due to these exposed hydrophobic amino acids (Fig. 6A). On the other hand, they considered that, in the second and following motifs, the 6th and 9th amino acids are buried inside the protein, and form a hydrophobic core, and therefore if hydrophilic residues are placed as the 6th and 9th amino acids of all the motifs, the protein structure may collapse. Therefore, they decided to decrease the aggregation property of PPR by using hydrophilic amino acid (asparagine, aspartic acid, glutamine, glutamic acid, lysine, arginine, serine, and threonine) as the 6th amino acid, preferably the 6th and 9th amino acids, in only the first motif.

Specific procedure is as follows.

In the first PPR motif (M₁) from the N-terminus of a protein capable of binding to a target nucleic acid having a specific nucleotide sequence:
(1) a hydrophilic amino acid is used as the A₆ amino acid, preferably asparagine or aspartic acid is used as the A₆ amino acid, and
(2) further, a hydrophilic amino acid or glycine, preferably glutamine, glutamic acid, lysine, or glycine, is used as the A₉ amino acid, or
(3) the A₆ amino acid and A₉ amino acid are constituted by any of the following combinations;
   - combination of asparagine as the A₆ amino acid and glutamic acid as the A₉ amino acid,
   - combination of asparagine as the A₆ amino acid and glutamine as the A₉ amino acid,
   - combination of asparagine as the A₆ amino acid and lysine as the A₉ amino acid, and
   - combination of aspartic acid as the A₆ amino acid and glycine as the A₉ amino acid.

Among such PPR motifs, the followings are particularly preferred:
(1st_A-1) a PPR motif consisting of the sequence of SEQ ID NO: 402 having such substitutions of the amino acids at positions 6 and 9 that any one of the combinations defined below is satisfied;
(1st_A-2) a PPR motif comprising the sequence of (1st_A-1) having a substitution, deletion, or addition of 1 to 9 amino acids other than the amino acids at positions 1, 4, 6, 9, and 34, and having an adenine-binding property;
(1st_A-3) a PPR motif having a sequence identity of at least 80% to the sequence of (1st_A-1), provided that the amino acids at positions 1, 4, 6, 9, and 34 are identical, and having an adenine-binding property;
(1st_C-1) a PPR motif consisting of the sequence of SEQ ID NO: 403;
(1st_C-2) a PPR motif comprising the sequence of (1st_C-1) having a substitution, deletion, or addition of 1 to 9 amino acids other than the amino acids at positions 1, 4, 6, 9, and 34, and having a cytosine-binding property;
(1st_C-3) a PPR motif having a sequence identity of at least 80% to the sequence of (1st_C-1), provided that the amino acids at positions 1, 4, 6, 9, and 34 are identical, and having a cytosine binding property;
(1st_G-1) a PPR motif consisting of the sequence of SEQ ID NO: 404 having such substitutions of the amino acids at positions 6 and 9 that any one of the combinations defined below is satisfied;
(1st_G-2) a PPR motif comprising the sequence of (1st_G-1) having a substitution, deletion, or addition of 1 to 9 amino acids other than the amino acids at positions 1, 4, 6, 9, and 34, and having a guanine-binding property;
(1st_G-3) a PPR motif having a sequence identity of at least 80% to the sequence of (1st_G-1), provided that the amino acids at positions 1, 4, 6, 9, and 34 are identical, and having a guanine-binding property;
(1st_U-1) a PPR motif consisting of the sequence of SEQ ID NO: 405 having such substitutions of the amino acids at positions 6 and 9 that any one of the combinations defined below is satisfied;
(1st_U-2) a PPR motif comprising the sequence of (1st_U-1) having a substitution, deletion, or addition of 1 to 9 amino acids other than the amino acids at positions 1, 4, 6, 9, and 34, and having a uracil-binding property; and
(1st_U-3) a PPR motif having a sequence identity of at least 80% to the sequence of (1st_U-1), provided that the amino acids at positions 1, 4, 6, 9, and 34 are identical, and having a uracil-binding property:
   - a combination of asparagine as the amino acid at position 6 and glutamic acid as the amino acid at position 9,
   - a combination of asparagine as the amino acid at position 6 and glutamine as the amino acid at position 9,
   - a combination of asparagine as the amino acid at position 6 and lysine as the amino acid at position 9,
   - a combination of aspartic acid as the amino acid at position 6 and glycine as the amino acid at position 9.

Fig. 6A shows the amino acid sequences of the v3.2 motifs as well as those of the v3.1 motifs. In v3.2, the first motif is selected from 1st_A (SEQ ID NO: 402), 1st_C (SEQ ID NO: 403), 1st_G (SEQ ID NO: 404), and 1st_U (SEQ ID NO: 405), and the second and the following motifs are selected from v2_C, v2_G, v2_U, and v3.1_A. The use of any of the v3.2 as the first PPR motif from the N-terminus in a PPR protein may improve intracellular aggregation property.

### (Others)

The term "identity" used in the present invention for base sequence (also referred to as nucleotide sequence) or amino acid sequence means percentage of number of matched bases or amino acids shared between two sequences aligned in an optimal manner, unless especially stated. In other words, the identity can be calculated in accordance with the equation: Identity = (Number of matched positions/Total number of positions) x 100, and it can be calculated by using commercially available algorithms. Such algorithms are also incorporated in the NBLAST and XBLAST programs described in Altschul et al., J. Mol. Biol., 215 (1990) 403-410. In more detail, the search and analysis for the identity of nucleotide or amino acid sequences can be performed with algorithms or programs well known to those skilled in the art (e.g., BLASTN, BLASTP, BLASTX, and ClustalW). In the case of using a program, parameters can be appropriately set by those skilled in the art, or the default parameters of each program can also be used. The specific procedures of these analysis methods are also well known to those skilled in the art.

In this description, when the identity is expressed as a percentage for a nucleotide sequence or amino acid sequence, a higher identity percentage value is preferred in both cases, unless especially stated, specifically, 70% or higher is preferred, 80% or higher is more preferred, 85% or higher is still more preferred, 90% or higher is further preferred, 95% or higher is still further preferred, and 97.5% or higher is even further preferred.

As for the term "sequence having a substitution, deletion, or addition" used in the present invention concerning PPR motif or protein, the number of amino acids substituted or the like is not particularly limited in any motif or protein, so long as the motif or protein comprising the amino acid sequence has the desired function, unless especially stated. The number of amino acids to be substituted, or the like may be about 1 to 9 or 1 to 4, or even larger number of amino acids may be substituted or the like if they are substituted with amino acids having similar properties. The means for preparing polynucleotides or proteins for such amino acid sequences are well known to those skilled in the art.

Amino acids having similar properties refer to amino acids with similar physical properties such as hydropathy, charge, pKa, and solubility, and refer to such amino acid as mentioned below, for example.

Hydrophobic (non-polar) amino acids; alanine, valine, glycine, isoleucine, leucine, phenylalanine, proline, tryptophan, tyrosine.

Non-hydrophobic amino acids; arginine, asparagine, aspartic acid, glutamic acid, glutamine, lysine, serine, threonine, cysteine, histidine, methionine.

Hydrophilic amino acids; arginine, asparagine, aspartic acid, glutamic acid, glutamine, lysine, serine, threonine.

Acidic amino acids: aspartic acid, glutamic acid.

Basic amino acids: lysine, arginine, histidine.

Neutral amino acids: alanine, asparagine, cysteine, glutamine, glycine, isoleucine, leucine, methionine, phenylalanine, proline, serine, threonine, tryptophan, tyrosine, valine.

Sulfur-containing amino acids: methionine, cysteine.

Aromatic ring-containing amino acids: tyrosine, tryptophan, phenylalanine.

The PPR motif, protein containing the same, or nucleic acids encoding the same of the present invention can be prepared by those skilled in the art using conventional techniques.

### [Performance of novel PPR motifs]

### (Binding power)

PPR proteins prepared by using the novel PPR motifs of the present invention (SEQ ID NOS: 9 to 12) are not only suitable for preparation of PPR proteins for relatively long target RNAs, but also may have higher RNA-binding performance compared with PPR proteins prepared by using existing PPR motifs (SEQ ID NOS: 13 to 16) for the same target RNA.

In other words, use of the novel PPR motifs of the present invention in a PPR protein can increase the binding power to a target RNA compared with use of the existing PPR motifs. By increasing the binding power, the efficiency of RNA manipulation using the PPR protein in the cell can be improved. For example, the efficiency of intracellular splicing can be improved by using a PPR protein showing high binding power to a target (see Example 5).

The degree of the improvement of the binding power is considered to vary depending on the sequence and length of the target, and the binding power can be enhanced, for example, 1.1 times or more, more specifically 1.3 times or more, 2.0 times or more, 3.0 times or more, or 3.6 times or more.

The binding power to a target sequence can be evaluated by EMSA (Electrophoretic Mobility Shift Assay) or a method using Biacore. EMSA is a method utilizing a property of nucleic acid that when a sample consisting of a nucleic acid bound with a protein is electrophoresed, the mobility of the nucleic acid molecule changes from that of the nucleic acid not bound. Molecular interaction analyzers, such as Biacore as a typical example, enable kinetic analysis, and therefore allow detailed protein-nucleic acid binding analysis.

The binding power to a target sequence can also be evaluated by RPB-ELISA described later. In RPB-ELISA, a value obtained by subtracting background signal (luminescence signal value obtained with an objective PPR protein without adding the target RNA) from luminescence obtained with a sample containing the objective PPR protein and the target RNA thereof can be used as the binding power of the objective PPR protein and the target RNA thereof.

### (Specificity)

A PPR protein prepared by using the novel PPR motifs of the present invention may have a higher capacity in specificity for a target sequence compared with a PPR protein prepared by using existing PPR motifs for the same target RNA.

That is, by using the novel PPR motifs of the present invention in the PPR protein, the specificity for the target RNA can be increased compared with the case of using existing PPR motifs. By using a PPR protein having higher specificity for a target RNA, unintended effects as a result of binding to an unintended RNA can be avoided, when the target RNA is manipulated in a cell using the PPR protein.

Affinity to a target sequence can be evaluated by conventional methods by those skilled in the art. In RPB-ELISA, by designing an appropriate non-target RNA for an objective PPR protein, and determining binding power for it (luminescence signal value) in the same manner, binding signal value for the target sequence/binding signal value for non-target sequence (S/N) can be determined as an index of specificity (affinity) for the target RNA.

### (Kd value)

The PPR protein prepared by using the novel PPR motifs of the present invention can have high affinity (equilibrium dissociation constant, Kd value) for a target RNA.

The Kd values for a target sequence can be calculated by existing methods such as EMSA. The Kd value used in the present invention refers to a value measured by EMSA under the conditions described in the section of Examples described below, unless especially stated.

Although the Kd value of a PPR protein prepared by using the novel PPR motifs of the present invention may be considered to depend on the sequence and length of the target, it can be 10⁻⁷ M or smaller, 10⁻⁸ M or smaller, or in the order of 10⁻⁹ M, when the length of the target sequence is 18 bases long or longer. According to the examination of the inventors of the present invention, when the length of the target sequence has 18-base long, the minimum Kd value (high affinity) was 1.95 × 10⁻⁹ under the conditions of the examples, which is lower than any of the previously reported Kd values of the designed PPR proteins (see Table 1). By the way, it has been revealed that the Kd values correlate with the signal values obtained in the RPB-ELISA binding experiments. It can be estimated that when the RPB-ELISA luminescence value (according to the conditions described in the Examples section) is 1 to 2 × 10⁷, the Kd value is 10⁻⁶ to 10⁻⁷ M, when the RPB-ELISA luminescence value is 2 to 4 × 10⁷, the Kd value is 10⁻⁷ to 10⁻⁸ M, and when the RPB-ELISA luminescence value is greater than 4 × 10⁷, the Kd value is 10⁻⁸ or smaller.

### (PPR protein construction efficiency)

By using the novel PPR motifs of the present invention, a desired PPR protein can be efficiently constructed. The construction efficiency can be calculated by determining the percentage of successful construction of PPR proteins with high Kd values using existing methods. The construction efficiency can also be calculated by using the luminescence signal value obtained by RPB-ELISA instead of the Kd value in the same manner as described above.

Specifically, when the length of the target sequence is 18 bases long, by using the novel PPR motif of the present invention, PPR proteins with a Kd value of 10⁻⁶ M or lower (RPB-ELISA value is 1 × 10⁷ or higher) can be obtained with an efficiency of 50% or higher, more specifically 60% or higher, still more specifically 70% or higher, further specifically 80% or higher. According to the present invention, PPR proteins with a Kd value of 10⁻⁷ M or lower (RPB-ELISA value is 2 × 10⁷ or higher) for a target sequence of 18 bases long can be obtained with an efficiency of 50% or higher, specifically 55% or higher, more specifically 65% or higher, further specifically 75% or higher. Further, according to the present invention, PPR proteins with a Kd value of 10⁻⁸ M or lower (RPB-ELISA value is 4 × 10⁷ or higher) for a target sequence of 18 bases long can be obtained with an efficiency of 20% or higher, specifically 25% or higher, more specifically 30% or higher, further specifically 35% or higher.

The construction efficiency can be calculated on the basis of the ratio of binding signal value to a target sequence/binding signal value to a non-target sequence (S/N) by using the RPB-ELISA method.

Specifically, by using the novel PPR motifs of the present invention, PPR proteins with an S/N of 10 or higher for a target sequence of 18 bases long can be obtained with an efficiency of 50% or higher, more specifically 55% or higher, still specifically 65% or higher, further specifically 75% or higher. According to the present invention, PPR proteins with an S/N of 100 or higher for a target sequence of 18 bases long can be obtained with an efficiency of 15% or higher, specifically 20% or higher, more specifically 25% or higher, further specifically 30% or higher.

### [Seamless cloning of PPR protein gene using parts library]

The present invention also provides a method for preparing a gene encoding a protein comprising n of PPR motifs that can bind to a target nucleic acid consisting of a sequence of n bases in length, which comprises the following steps of:
selecting m of PPR parts required to prepare the objective gene from a library of at least 20 × m kinds of PPR parts, which consist of m kinds of intermediate vectors Dest-a, ... , which are designed so that they can successively bind, and are inserted with at least 20 kinds of polynucleotides including 4 kinds encoding PPR motifs that have adenine, cytosine-, guanine-, uracil- and thymine-binding properties, respectively, and 16 kinds of the same encoding linkage products of two of the PPR motifs, respectively; and
subjecting the selected m kinds of PPR parts to the Golden Gate reaction together with the vector parts to obtain a vector in which m of polynucleotide linkage products are inserted. n is an integer of m or larger, and is m × 2 or smaller. n can be, for example, 10 to 20.

The method of the present invention utilizes the Golden Gate reaction. In the Golden Gate reaction, multiple DNA fragments are inserted into a vector using a type IIS restriction enzyme and T4 DNA ligase. The type IIS restriction enzyme cleaves a nucleic acid at a position outside the recognition sequence, and therefore the cohesive end can be freely chosen. Further, since it uses 4 bases protruding end for the ligation, it is highly efficient. Furthermore, the recognition sequence does not remain in the construct obtained after annealing and ligation. Therefore, polynucleotides encoding PPR motifs can be seamlessly ligated (Fig. 5). A particularly preferred example of the type IIS restriction enzyme is BsaI.

The method of the present invention enables efficient preparation of a gene by using a parts library appropriately designed in consideration of the characteristics of the PPR proteins and the Golden Gate reaction, even when the gene contains a large number of repeat sequences. Therefore, this method is useful for preparing a gene of a protein containing 15 or more of PPR motifs that can bind to a target nucleic acid of 15 base length or longer, which requires a larger number of repeat sequences. When m is 10, by selecting 10 of PPR parts necessary for preparing a target gene from a library consisting of 200 PPR parts, a gene encoding a protein containing 10 to 20 PPR motifs can be prepared as desired. In the following descriptions, explanations may be made by exemplifying preparation of a gene encoding an RNA-binding PPR protein whose target sequence has 10 to 20 bases long. However, this method can also be applied to preparation of a PPR protein for a target sequence of a different length, and it can also be applied to preparation of a DNA-binding PPR protein.

In the method of the present invention, a library of parts comprising one or two sequences encoding a PPR motif is prepared (STEP 1 and STEP 2 in Fig. 5), and used. The parts library can be prepared by, for example, inserting the PPR motif sequences into 10 different intermediate vectors Dest-a, b, c, d, e, f, g, h, i, and j. The intermediate vectors are designed so that Dest-a to Dest-j are successively and seamlessly ligated by the Golden Gate reaction. The PPR motif sequences to be inserted may consist of at least 20 kinds of sequences including 4 kinds encoding each base (A, C, G, and U) and 16 kinds encoding each of the ligation products of two of the PPR motifs (AA, AC, AG, AU, CA, CC, CG, CU, GA, GC, GG, GU, UA, UC, UG, and UU). In this case, the parts library comprises at least 200 types of parts.

Then, necessary parts are selected according to the target nucleotide sequence. Specifically, for example, one part each is selected from each of the Dest-a, b, c, d, e, f, g, h, i, and j parts libraries, and subjected to the Golden Gate reaction together with the vector parts (STEP 3 in Fig. 5). If an intermediate vector containing 1 motif is selected for all the intermediate vectors, 10 sequences are ligated, or if intermediate vectors containing 2 motifs are used, 20 sequences are ligated. When it is desired to link 11 to 19 sequences, 1 motif can be selected from each of any Dest-x libraries.

The vector parts to be used in STEP 3 can be selected from three types of CAP-x vectors (consideration for the ii-th amino acid in the PPR motif closest to the C-terminus is required, but the ii-th amino acids of the guanine-binding PPR motif and the uracil-binding PPR motif are identical, see Non-patent document 1 mentioned above). If the base recognized by the motif closest to the C-terminus is adenine, CAP-A can be used, if it is cytosine, CAP-C can be used, and if it is guanine or uracil, CAP-GU can be used.

The resulting plasmids can be transformed into *E. coli,* then amplified and extracted.

### [Method for detection or analysis of PPR protein]

The present invention provides a method for detecting or quantifying a protein comprising n of PPR motifs that can bind to a target nucleic acid consisting of a sequence of n bases in length, which comprises the following steps:
the step of adding a solution containing a candidate protein to a solid-phased target nucleic acid, and detecting or quantifying the protein that bound to the target nucleic acid.

This detection or analysis method of the present invention is useful as a high throughput method for evaluating binding performance of PPR proteins.

Since the detection or analysis method of the present invention is based on the application of ELISA (Enzyme-Linked Immuno Sorbent Assay) (Fig. 7A), it may be referred to as RPB-ELISA (RNA-protein binding ELISA) method. Although the method is described herein as a method for evaluating RNA-binding PPR proteins, it can also be applied to evaluation of the binding performance of DNA-binding PPR proteins to a target DNA.

The step of adding a solution containing a candidate protein to a solid-phased target nucleic acid can be specifically carried out by flowing a solution containing the objective binding protein on the target nucleic acid molecule immobilized on a plate. Immobilization of the target nucleic acid molecule can be achieved by using various existing immobilization methods, such as by providing a nucleic acid probe containing a biotin-modified target nucleic acid molecule to a streptavidin-coated well plate.

On the other hand, the candidate protein to be measured can be fused with a marker protein, for example, an enzyme such as luciferase or a fluorescent protein. The fusion with the marker protein makes the detection and quantification easier.

The RPB-ELISA method has an advantage that it does not require special equipment such as Biacore. In addition, the RPB-ELISA method provides high throughput, and enables evaluation of binding between protein and nucleic acid in a short time. Furthermore, the RPB-ELISA method has an advantage that it enables sufficient detection at a protein concentration of 6.25 nM or higher under the conditions used in the examples, and similarly enables detection also with *E. coli* lysates, and therefore it does not require purification of the target nucleic acid-binding protein.

### [Use of PPR protein]

### (Complex and fusion protein)

The PPR motif or PPR protein provided by the present invention can be made into a complex by binding a functional region. The PPR motif or PPR protein can also be linked with a proteinaceous functional region to form a fusion protein. The functional region refers to a part having such a function as a specific biological function exerted in a living body or cell, for example, enzymatic function, catalytic function, inhibitory function, promotion function, etc, or a function as a marker. Such a region consists of, for example, a protein, peptide, nucleic acid, physiologically active substance, or drug. In the following explanations, the complex of the present invention may be explained with reference to a fusion protein as an example, but those skilled in the art may also understand complexes other than fusion protein according to the explanations.

In one of the preferred embodiments, the functional region is a ribonuclease (RNase). Examples of RNase are RNase A (e.g., bovine pancreatic ribonuclease A, PDB 2AAS), and RNase H.

In one of the preferred embodiments, the functional region is a fluorescent protein. Examples of fluorescent protein are mCherry, EGFP, GFP, Sirius, EBFP, ECFP, mTurquoise, TagCFP, AmCyan, mTFP1, MidoriishiCyan, CFP, TurboGFP, AcGFP, TagGFP, Azami-Green, ZsGreen, EmGFP, HyPer, TagYFP, EYFP, Venus, YFP, PhiYFP, PhiYFP-m, TurboYFP, ZsYellow, mBanana, KusabiraOrange, mOrange, TurboRFP, DsRed-Express, DsRed2, TagRFP, DsRed-Monomer, AsRed2, mStrawberry, TurboFP602, mRFP1, JRed, KillerRed, HcRed, KeimaRed, mRasberry, mPlum, PS-CFP, Dendra2, Kaede, EosFP, and KikumeGR. A preferred example is mClover3 in view of improvement of aggregation and/or efficient localization to the nuclei as a fusion protein.

In one of the preferred embodiments, when the target is mRNA, the functional region is a functional domain that enhances expression amount of a protein from the target mRNA (WO2017/209122). The functional domain that enhances expression amount of a protein from mRNA may be, for example, all or a functional part of a functional domain of a protein known to directly or indirectly promote translation of mRNA. More specifically, it may be a domain that directs ribosomes to mRNA, domain associated with initiating or promoting translation of mRNA, domain associated with transporting mRNA out of the nucleus, domain associated with binding to the endoplasmic reticulum membrane, domain containing an endoplasmic reticulum (ER) retention signal sequence, or domain containing an endoplasmic reticulum signal sequence. More specifically, the domain that directs ribosomes to mRNA mentioned above may be a domain comprising all or a functional part of a polypeptide selected from the group consisting of density-regulated protein (DENR), malignant T-cell amplified sequence 1 (MCT-1), transcriptionally-controlled tumor protein (TPT1), and Lerepo4 (zinc finger CCCH-domain). The domain associated with translation initiation or translation promotion of mRNA mentioned above may be a domain comprising all or a functional part of a polypeptide selected from the group consisting of eIF4E and eIF4G. The domain associated with transporting mRNA out of the nucleus mentioned above may be a domain containing all or a functional part of stem-loop binding protein (SLBP). The domain associated with binding to the endoplasmic reticulum membrane mentioned above may be a domain comprising all or a functional part of a polypeptide selected from the group consisting of SEC61B, translocation associated protein alpha (TRAP-alpha), SR-alpha, Dial (cytochrome b5 reductase 3), and p180. The endoplasmic reticulum retention signal (ER retention signal) sequence mentioned above may be a signal sequence comprising the KDEL (KEEL) sequence. The endoplasmic reticulum signal sequence mentioned above may be a signal sequence including MGWSCIILFLVATATGAHS.

In the present invention, the functional region may be fused to the PPR protein on the N-terminal side or the C-terminal side, or on both the N-terminal side and the C-terminal side. The complex or fusion protein may include a plurality of functional regions (e.g., 2 to 5). Further, the complex or fusion protein according to the present invention may consist of the functional region and PPR protein indirectly fused via a linker or the like.

### (Nucleic acid encoding PPR protein etc., vector, and cell)

The present invention also provides a nucleic acid encoding the PPR motif, PPR protein or fusion protein mentioned above, and a vector containing such a nucleic acid (e.g., vector for amplification, and expression vector). As the host of the vector for amplification, *E. coli* or yeast may be used. In this description, expression vector means a vector containing, for example, a DNA having a promoter sequence, DNA encoding a desired protein, and DNA having a terminator sequence from the upstream side, but they need not necessarily be arranged in this order, so long as the desired function is exerted. In the present invention, recombinant vectors prepared by using various vectors that may be normally used by those skilled in the art may be used.

The PPR protein or fusion protein of the present invention can function in eukaryotic (e.g., animal, plant, microbe (yeast, etc.), and protozoan) cells. The fusion protein of the present invention can function, in particular, in animal cells (in vitro or in vivo). Examples of animal cells into which the PPR protein or fusion protein of the present invention, or a vector expressing it can be introduced include, for example, cells derived from humans, monkeys, pigs, cows, horses, dogs, cats, mice, and rats. Examples of cultured cells into which the PPR protein or fusion protein of the present invention or a vector expressing it can be introduced include, for example, Chinese hamster ovary (CHO) cells, COS-1 cells, COS-7 cells, VERO (ATCC CCL-81) cells, BHK cells, canine kidney-derived MDCK cells, hamster AV-12-664 cells, HeLa cells, WI38 cells, 293 cells, 293T cells, and PER.C6 cells, but not limited to these.

### (Use)

With the PPR protein or fusion protein of the present invention, a functional region may be delivered to the inside of a living body or cells and made to function in a nucleic acid sequence-specific manner. A complex linked with a marker such as GFP may be used to visualize a desired RNA in a living body.

With the PPR protein or fusion protein of the present invention, a nucleic acid can be modified or disrupted in a nucleic acid sequence-specific manner in the inside of cells or living bodies, and a new function may be conferred. In particular, RNA-binding PPR proteins are involved in all the RNA processing steps found in the organelles, such as cleavage, RNA edition, translation, splicing, and RNA stabilization. Accordingly, such uses of the method concerning modification of PPR proteins provided by the present invention, as well as the PPR motif and PPR protein provided by the present invention as mentioned below can be expected in a variety of fields.

### (1) Medical care

- Creation of a PPR protein that recognizes and binds to a specific RNA associated with a specific disease. Analysis of a target sequence and associated proteins for a specific RNA. The results of the analysis can be used to identify compounds for the treatment of the disease.

For example, it is known that, in animals, abnormalities in the PPR protein identified as LRPPRC cause Leigh syndrome, French Canadian type (LSFC, Leigh syndrome, subacute necrotizing encephalomyelopathy). The present invention may contribute to the treatment (prevention, therapeutic treatment, or inhibition of progression) of LSFC. Many of the existing PPR proteins work to specify edition sites for RNA manipulation (conversion of genetic information on RNA, often C to U). The PPR proteins of this type have an additional motif that is suggested to interact with RNA editing enzymes on the C-terminal side. PPR proteins having this structure are expected to enable introduction of base polymorphism or treatment of a disease or condition caused by base polymorphism.
- Creation of cells with controlled RNA repression/expression. Such cells include stem cells of which differentiation or undifferentiation state is monitored (e.g., iPS cells), model cells for evaluation of cosmetics, and cells in which the expression of functional RNA can be turned on or off for the purpose of elucidating action mechanism and pharmacological testing for drug discovery.
- Preparation of a PPR protein that specifically binds to a specific RNA associated with a particular disease. Such a PPR protein is introduced into a cell using a plasmid, virus vector, mRNA, or purified protein, and an RNA function that causes a disease can be changed (improved) by binding of the PPR protein to the target RNA in the cell. Examples of the mechanism of changing the function include, for example, change of the RNA structure by binding, knockdown by decomposition, change of the splicing reaction by splicing, base substitution, and so forth.

### (2) Agriculture, forestry and fishery

- Improvement of yield and quality of crops, forest products and marine products.
- Breeding of organisms with improved disease resistance, improved environmental tolerance, or improved or new function.

For example, concerning hybrid firstgeneration (F1) plant crops, an F1 plant may be artificially created by using stabilization of mitochondrial RNA and translation control by PPR proteins so that yield and quality of the crops may be improved. RNA manipulation and genome edition using PPR proteins more accurately and quickly enable variety improvement and breeding (genetic improvement of organisms) of organisms compared with conventional techniques. In addition, it can be said that RNA manipulation and genome editing using PPR proteins are similar to the classical breeding methods such as selection of mutants and backcrossing, since they do not transform traits with a foreign gene as in genetic recombination, but they are techniques using RNA and genomes originally possessed by plants and animals. Therefore, they can also surely and quickly cope with global-scale food and environmental problems.

### (3) Chemistry

- Control of protein expression amount by manipulating DNA and RNA in the production of useful substances using microorganisms, cultured cells, plant bodies, and animal bodies (e.g., insect bodies). Productivity of useful substances can be thereby improved. Examples of the useful substances are proteinaceous substances such as antibodies, vaccines, and enzymes, as well as relatively low-molecular weight compounds such as pharmaceutical intermediates, fragrances, and dyes.
- Improvement of production efficiency of biofuel by modification of metabolic pathways of algae and microorganisms.

### Examples

### [Example 1: Establishment of method for preparing PPR gene]

### (Design of motif)

First, PPR motifs were designed. For the PPR motif sequences used in the artificial PPR proteins reported so far, consensus sequences of naturally occurring PPR motif sequences extracted by various methods were used. Among them, PPR proteins made from the motif sequence of dPPR (Non-patent documents 2, 3, and 6 mentioned above) have a low Kd value (high affinity). This PPR motif sequence is hereinafter referred to as v1 PPR motif.

As for the other PPR motif sequences, there were prepared consensus sequences by using only PPR motifs containing representative combinations of 1st, 4th, and ii-nd amino acid that recognize each base. Specifically, the representative amino acid combinations that recognize each base are: combination of first valine, fourth threonine, and ii-th asparagine that recognizes adenine, combination of first valine, fourth asparagine, and ii-th serine that recognizes cytosine, combination of first valine, fourth threonine, and ii-th aspartic acid that recognizes guanine, and combination of first valine, fourth asparagine, and ii-th aspartic acid that recognizes uracil, therefore consensus amino acid sequences were extracted from the PPR motif sequences containing those combinations of the first, fourth, and ii-th amino acids, and these sequence were used as PPR motif sequences that specifically recognizes adenine, cytosine, guanine, and uracil, respectively (Figs. 1 to 4, SEQ ID NOS: 9 to 12). These motifs will be henceforth referred to as v2 PPR motifs. The same combinations of 1st, 4th, and ii-th amino acids were also used for the v1 PPR motifs (SEQ ID NOS: 13 to 16).

### (Seamless cloning using one- and two-motif libraries)

A cloning method for seamlessly ligating these designed PPR motif sequences was constructed (Fig. 5). The cloning is performed through three steps. In STEP 1, the motif sequences are designed and prepared. In STEP 2, plasmid libraries in which one or two motifs are cloned are prepared. In STEP 3, required number of the motifs are ligated to complete a target PPR gene.

First, plasmids in which one PPR motif sequence (numbers 4 to ii) was cloned were prepared (STEP 1). The plasmids of STEP 1 contained PPR motif sequences recognizing A, C, G, and U, respectively. In the following STEP 2, DNA fragments containing the PPR motif sequence in the plasmids of STEP 1 were cloned into an intermediate vector (Dest-x, the sequence thereof is shown below). The plasmids of STEP 1 that enable insertion of one motifs were designated as P1a-vx-X, and as for the plasmids that enable insertion of two motifs, those of the N-terminus side were designated as P2a-vx-X, and those of C-terminus side as P2b-vx-X (vx is v1 or v2, and X is A, C, G, or U). For cloning into Dest-x, the BsaI restriction enzyme site (BsaI restriction enzyme recognizes and cleaves the sequence GGTCTCnXXXX (SEQ ID NO: 17), where the XXXX portion constitutes a four bases protruding end (henceforth referred to as tag sequence). The sequences to be seamlessly ligated were designed as follows.

There were prepared nucleotide sequences comprising each motif sequence, and the following sequences ligated on the 5' and 3' sides of the motif sequence: ggtctcaatac (SEQ ID NO: 18), and gtggtgagacc (SEQ ID NO: 19) in the case of P1a, ggtctcaatac (SEQ ID NO: 18 mentioned above), and gtggtcacatatgagacc (SEQ ID NO: 20) in the case of P2a, or ggtctcacatac (SEQ ID NO: 21), and gtggtgagacc (SEQ ID NO: 19 mentioned above) in the case of P2b, by a gene synthesis technique, and they were cloned into pUC57-amp.

There were 10 types of Dest-x (Dest-a, b, c, d, e, f, g, h, i, and j), and the sequences thereof were designed so that Dest-a to Dest-j could be seamlessly ligated in that order.

There were prepared gaagacataaactccgtggtcacATACagagaccaaggtctcaGTGGtcacatacatgtcttc (SEQ ID NO: 1) as Dest-a, gaagacatATACagagaccaaggtctcaGTGGtgacataatgtcttc (SEQ ID NO: 22) as Dest-b, gaagacatcATACagagaccaaggtctcaGTGGttacatatgtcttc (SEQ ID NO: 23) as Dest-c, gaagacatacATACagagaccaaggtctcaGTGGttacaatgtcttc (SEQ ID NO: 24) as Dest-d, gaagacattacATACagagaccaaggtctcaGTGGtgacatgtcttc (SEQ ID NO: 25) as Dest-e, gaagacattgacATACagagaccaaggtctcaGTGGttaatgtcttc (SEQ ID NO: 26) as Dest-f, gaagacatgttacATACagagaccaaggtctcaGTGGtcatgtcttc (SEQ ID NO: 27) as Dest-g, gaagacatggtcacATACagagaccaaggtctcaGTGGtatgtcttc (SEQ ID NO: 28) as Dest-h, gaagacattggttacATACagagaccaaggtctcaGTGGatgtcttc (SEQ ID NO: 29) as Dest-i, and gaagacatgtggtgacATACagagaccaaggtctcaGTGGtcttc (SEQ ID NO: 30) as Dest-j by a gene synthesis technique, and cloned into pUC57-kan.

Plasmids consisting of each Dest-x into which PPR motif corresponding to A, C, G, or U, or two PPR motifs that recognizes each of the base combination of AA, AC, AG, AU, CA, CC, CG, CU, GA, GC, GG, GU, UA, UC, UG, and UU were inserted were prepared for all of Dest-x to prepare plasmid libraries of STEP 1 for v1 and v2 (each comprises 200 types). In each of the combinations mentioned above, 40 ng of P1a plasmid alone, or 40 ng of P2a plasmid and 40 ng of P2b plasmid were combined with 0.2 µL of 10 x ligase buffer (NEB, B0202S), 0.1 µL of BsaI (NEB, R0535S), and 0.1 µL of Quick ligase (NEB, M2200S), and the total volume was adjusted to 1.9 µL with sterile water. Reactions were allowed at 37°C for 5 minutes and 16°C for 5 minutes, which were alternately repeated for 5 cycles, in a thermal cycler (Biorad, 1861096J1). Further, 0.1 µL of 10x Cut smart buffer (NEB, B7204) and 0.1 µL of Bsal (NEB, R0535S) were added, and reactions were allowed at 37°C for 60 minutes and 80°C for 10 minutes. XL1-blue was transformed with 2.5 µL of the reaction solution, and selected in the LB medium containing 30 µg/ml of kanamycin. Insertion of the desired sequences was confirmed by sequencing.

In STEP 3, Dest-a to Dest-j were selected according to the target sequence, and cloned into the CAP-x vector (Non-patent document 1 mentioned above). If those each containing one motif are used for all the intermediate vectors, 10 motifs are ligated, and if those containing two motifs each are used, 20 motifs are ligated. A plasmid comprising 11 to 19 motifs can be obtained by using Dest-x containing one motif at any position. For example, when an 18-motif PPR sequence is prepared, Dest-a and Dest-b containing one motif in, and the other plasmids containing two motifs are used.

The intermediate vectors used in the cloning of STEP 3 should be selected from three types of vectors. There were used CAP-A when the nucleotide to be recognized by the motif nearest to the C-terminus is adenine; CAP-C, when the same is cytosine; and CAP-GU, when the same is guanine or uracil. They were designed so that the amino acid sequence MGNSV (SEQ ID NO: 31) was added on the N-terminus side of the PPR repeat, and ELTYNTLISGLGKAGRARDPPV (SEQ ID NO: 32) was added on the C-terminus side of the PPR repeat as a result of the cloning of them into the intermediate vectors for STEP 3.

Each of 10 kinds of the intermediate plasmids in an amount of 20 ng, 1 µL of 10 x ligase buffer (NEB, B0202S), 0.5 µL of BpiL (Thermo, ER1012), and 0.5 µL of Quick ligase (NEB, M2200S) were combined, and the final volume was adjusted to 10 µl with sterile water. Reactions were allowed at 37°C for 5 minutes and 16°C for 7 minutes for 15 cycles. Further, 0.4 µL of BpiL was added, and reactions were allowed at 37°C for 30 minutes and 75°C for 6 minutes. Subsequently, 0.3 µL of 1 mM ATP and 0.15 µL of Plasmid safe nuclease (Epicentre, E3110K) were added, and reaction was allowed at 37°C for 15 minutes. *E. coli* (competent cells of XL-1 Blue strain, Nippon Gene) was transformed with 3.5 µl of the reaction solution, and cultured in the LB medium containing 100 µg/mL spectinomycin at 37°C for 16 hours for selection. A portion of the generated colonies was used to amplify the inserted gene region using primers pCR8_Fw: 5'-TTGATGCCTGGCAGTTCCCT-3' (SEQ ID NO: 33) and pCR8_Rv: 5'-CGAACCGAACAGGCTTATGT-3' (SEQ ID NO: 34). To a 0.2-mL tube, 5 µL 2 x Go-taq (Promega, M7123), 1.5 µL of 10 µM pCR8_Fw, 1.5 µL of 10 µM pCR8_Rv, and 2 µL of sterile water were added, and reaction was allowed at 98°C for 2 minutes, followed by 15 cycles of 98°C for 5 seconds, 55°C for 10 seconds, and 72°C for 2.5 minutes in a thermal cycler to carry out the DNA amplification reaction. A portion of the reaction solution was electrophoresed by using MultiNA (SHIMADZU, MCE202) to confirm the size of the inserted DNA fragment. By using v1 and v2 motifs, three clones were prepare for each of the three kinds of 18-motif PPR proteins (PPR1, PPR2, and PPR3, SEQ ID NOS: 35 to 37 and, 40 to 42) (v1_PPR1, vl_PPR2, v1_PPR3, v2_PPR1, v2_PPR2, and v2_PPR3), of which results are shown in Fig. 6B. With v1, bands of correct size were obtained except for the second clone of PPR2. With v2, bands of correct size were obtained for all the clones. In addition, the sequences of them were confirmed by sequencing. These results indicate that the PPR protein genes can be efficiently constructed by cloning according to this method.

### [Example 2: Construction of high throughput binding performance evaluation system for RNA-binding protein]

In general, evaluation of binding between a nucleic acid-binding protein and a nucleic acid molecule is performed by a method using EMSA or Biacore. EMSA (Electrophoretic Mobility Shift Assay) is a method utilizing the property that when a sample of a protein and a nucleic acid bound together is electrophoresed, mobility of the nucleic acid molecule changes compared with that of the molecule not bound. This method has drawbacks that it requires purified protein, operation is complicated, and it cannot analyze a large number of samples at one time. Molecular interaction analyzers, of which typical example is Biacore, enable reaction kinetics analyses, and therefore allow detailed protein-nucleic acid binding analysis. However, they also require purified protein and special equipment. Therefore, the inventors thought of a method that enables evaluation of protein-nucleic acid binding in a short time with high throughput.

ELISA (Enzyme-Linked Immuno Sorbent Assay) is generally used to analyze binding of antibody (protein) and protein. In this method, a primary antibody is fixed on a well plate, to which a solution containing a protein to be detected is added, and after washing, a secondary antibody detectable with color development or luminescence is added, and quantified in order to detect the amount of the remaining protein as the object of the analysis. By applying this method, the inventors devised a system, in which a nucleic acid molecule is fixed on a plate, a solution containing an objective nucleic acid-binding protein is poured onto the plate, and the amount of bound protein is quantified (Fig. 7A). The nucleic acid to be analyzed is fixed by adding a nucleic acid probe consisting of the nucleic acid having a biotin-modified end to a streptavidin-coated well plate. By fusing the nucleic acid-binding protein to be analyzed to luciferase or fluorescent protein, detection can be made easier. In addition, the protein to be measured may not necessarily be purified, and the analysis can be performed by using a crude extract of cells, in which the nucleic acid-binding protein to be measured is expressed (cultured animal cells, yeast, *E. coli,* etc.), and the time for purification can be thereby shortened (Fig. 7B). This method used for measuring binding of RNA and RNA-binding protein is henceforth referred to as RPB-ELISA (RNA-protein binding ELISA).

To establish the experiment system, a recombinant MS2 protein and an RNA probe that binds to it were prepared. A gene for the MS2 protein fused with luciferase protein on the N-terminus side and 6x histidine tag on the C-terminus side was prepared by gene synthesis, and cloned into the pET21b vector (NL_MS 2_HIS, SEQ ID NO: 357). As the RNA probe, a target sequence containing the MS2 binding sequence (RNA_4, SEQ ID NO: 64) and a non-target sequence not containing the MS2 binding sequence (RNA_51, SEQ ID NO: 247), each of which had 5'-end modified by biotinylation, were synthesized (Greiner). The Rosetta (DE3) strain of *E. coli* was transformed with the MS2 protein expression plasmid, and cultured overnight at 37°C in 2 mL of the LB medium containing 100 µg/mL ampicillin. Then, 2 mL of the culture medium was added to 300 mL of the LB medium containing 100 µg/mL ampicillin, and cultured at 37°C until OD₆₀₀ reached 0.5 to 0.8. After the temperature of the medium containing the cultured cells was lowered to 15°C, IPTG was added at a final concentration of 0.1 mM, and the culture was further continued for 12 hours. The culture medium was centrifuged at 5000 x g and 4°C for 10 minutes to collect the cells, 5 mL of a lysis buffer (20 mM Tris-HCl, pH 8.0, 150 mM NaCl, 0.5% NP-40, 1 mM DTT, 1 mM EDTA) was added, the mixture was stirred with a vortex mixer, and the cells were disrupted by sonication. The sonicated mixture was centrifuged at 15,000 rpm and 4°C for 10 minutes, and the supernatant was collected. Half of the supernatant was stored at -80°C until use as *E. coli* lysate, and the rest was affinity-purified using histidine tag and Ni-NTA. First, 200 µl of Ni-NTA agarose beads (Qiagen, Cat. No. 30230) were spun down, and the beads were collected. The beads were equilibrated by adding 100 µL of a washing buffer, and stirring the mixture on a rotator at 4°C for 1 hour. The entire volume of the equilibrated beads was mixed with the protein solution, and reaction was allowed at 4°C for 1 hour. Then, the beads were collected by centrifugation at 2,000 rpm for 2 minutes, and washed with 10 ml of a washing buffer (20 mM Tris-HCl, pH 8.0, 500 mM NaCl, 0.5% NP-40, 10 mM imidazole) to remove factors that nonspecifically bound to the beads. Elution was performed with 60 µL of an elution buffer (20 mM Tris-HCl, pH 8.0, 500 mM NaCl, 0.5% NP-40, 500 mM imidazole). Purification degree was confirmed by SDS-PAGE. The eluate was dialyzed overnight at 4°C against 20 mM Tris-HCl, pH 8.0, 150 mM NaCl, 0.5% NP-40, 1 mM DTT, 1 mM EDTA.

The luciferase luminescences of the *E. coli* lysate and the purified MS2 protein solution were measured. To a 96-well white plate, 40 µL of luciferase substrate (Promega, E151A) diluted 2500-fold with a luminescence buffer (20 mM Tris-HCl, pH 7.6, 150 mM NaCl, 5 mM MgCl₂, 0.5% NP-40, 1 mM DTT), and 40 µL of the *E. coli* lysate or 40 µL of the purified MS2 protein solution were added, and allowed to react for 5 minutes, after which the luminescence was measured with a plate reader (PerkinElmer, 5103-35). On the basis of the obtained luminescences, they were diluted with a lysis buffer (20 mM Tris-HCl, pH 7.6, 150 mM NaCl, 5 mM MgCl₂, 0.5% NP-40, 1 mM DTT, 0.1% BSA) to obtain dilution products of 0.01 × 10⁸, 0.02 × 10⁸, 0.09 × 10⁸, 0.38 × 10⁸, 1.50 × 10⁸, and 6.00 × 10⁸ LU/µL.

To a 96-well streptavidin-coated white plate (Thermo fisher, 15502), 2.5 pmol of the biotinylated RNA probe was added, reaction was allowed at room temperature for 30 minutes, and the plate was washed with the lysis buffer. For the background measurement, wells to which the biotinylated RNA was not added, but the lysis buffer was added (-Probe) were also prepared. Then, a blocking buffer (20 mM Tris-HCl, pH 7.6, 150 mM NaCl, 5 mM MgCl₂, 0.5% NP-40, 1 mM DTT, 1% BSA) was added, and the plate surface was blocked at room temperature for 30 minutes. Then, 100 µL of the *E. coli* lysate or purified protein solution diluted above was added to each well, and the binding reaction was allowed at room temperature for 30 minutes. Then, the wells were washed 5 times with 200 µL of a washing buffer (20 mM Tris-HCl, pH 7.6, 150 mM NaCl, 5 mM MgCl₂, 0.5% NP-40, 1 mM DTT). To each well, 40 µL of the luciferase substrate (Promega, E151A) diluted 2,500-fold with the washing buffer was added, reaction was allowed for 5 minutes, and then the luminescence was measured with a plate reader (PerkinElmer, 5103-35).

The background (luminescence signal value obtained with adding the PPR protein and without adding RNA) was subtracted from the luminescences of the samples to which a solution containing each of the RNA and MS2 protein was added, and the obtained values were used as the binding powers between the MS2 protein and RNA.

The results are shown in Fig. 7C. Specific binding of the MS2 protein to the target RNA (Target seq.) was detected for both the purified protein solution (Purified protein) and *E. coli* lysate (Lysate). The luminescence of 6.0 × 10⁸ LU/µL corresponds to 100 nM purified MS2 protein, and therefore it was found that detection can be sufficiently attained with a protein concentration of 6.25 nM (0.38 × 10⁸ LU/µL) or higher. Furthermore, the detection was also possible with the *E. coli* lysate, and therefore it was found that purification of the protein is not required.

### [Example 3: RNA binding performance comparison experiment for 18-motif PPR proteins prepared by using existing or novel PPR motif sequences]

In order to evaluate the RNA-binding performance of the PPR proteins prepared by using v1 or v2 PPR motifs, recombinant proteins were prepared in *E. coli,* and the binding performance thereof was evaluated by using RPB-ELISA. For the comparison, 5 kinds of target sequences (T_1, T_2, T_3, T 4, and T_5, SEQ ID NOS: 46 to 50) were determined, PPR proteins binding to each of them were designed, and genes encoding them were prepared (v1_PPR1, v1_PPR2, v1_PPR3, v1_PPR4, v1_PPR5, v2_PPR 1, v2_PPR2, v2_PPR3, v2_PPR4, and v2_PPR5, SEQ ID NOS: 35 to 39, and 40 to 45). To each prepared PPR gene, the luciferase protein gene was added on the N-terminus side, and a his-tag sequence was added on the C-terminus side, and they were cloned into the pET21 vector (NL_v1_PPR1, NL_v1_PPR2, NL_v1_PPR3, NL_v1_PPR4, NL-v1-PPR5, NL_v2_PPR1, NL_v2_PPR2, NL_v2_PPR3, NL_v2_PPR4, and NL_v2_PPR5, SEQ ID NOS: 51 to 60). The Rosetta (DE3) strain was transformed with the PPR expression plasmids. The *E. coli* was cultured in 2 mL of the LB medium containing 100 µg/mL ampicillin at 37°C for 12 hours. When OD₆₀₀ reached 0.5 to 0.8, the culture medium was transferred to an incubator at 15°C, and left standing for 30 minutes. Then, 100 µL of an IPTG solution was added (IPTG final concentration, 0.1 mM), and the culture was further continued at 15°C for 16 hours. An *E. coli* pellet was collected by centrifugation at 5,000 × g and 4 °C for 10 minutes, 1.5 mL of a lysis buffer (20 mM Tris-HCl, pH 8.0, 150 mM NaCl, 0.5% NP-40, 1 mM MgCl₂, 2 mg/mL lysozyme, 1 mM PMSF, 2 µL of 10 mg/mL DNase) was added to the pellet, and the mixture was frozen at -80°C for 20 minutes. The cells were cryodisrupted with permeabilization at 25°C for 30 minutes. The disrupted cell mixture was then centrifuged at 3,700 rpm and 4°C for 15 minutes, and the supernatant containing soluble PPR protein (*E. coli* lysate) was collected.

RNA probes consisting of a designed 30-base sequence containing 18 bases of the target sequence and modified by biotinylation at the 5' end (RNA_1, RNA_2, RNA_3, RNA_4, and RNA_5, SEQ ID NOS: 61 to 65) were synthesized (Grainer). To a streptavidin-coated plate (Thermo fisher), the 5'-end biotinylated RNA probes were added, reaction was allowed at room temperature for 30 minutes, and the plate was washed with a lysis buffer (20 mM Tris-HCl, pH 7.6, 150 mM NaCl, 5 mM MgCl₂, 0.5% NP -40, 1 mM DTT, 0.1% BSA). For background measurement, wells to which RNA was not added, but 100 µL of the lysis buffer, 1 µL of 100 mM DTT, and 1 µL of 40 unit/µL RNase inhibitor (Takara, 2313A) were added were also prepared. Then, 200 µL of a blocking buffer (20 mM Tris-HCl, pH 7.6, 150 mM NaCl, 5 mM MgCl₂, 0.5% NP-40, 1 mM DTT, 1% BSA) was added, and the plate surface was blocked at room temperature for 30 minutes. Then, 100 µL of *E. coli* lysate containing luciferase-fused PPR protein having a luminescence level of 1.5 × 10⁸ LU/µL was added to each well, and the binding reaction was allowed at room temperature for 30 minutes. The well was washed 5 times with 200 µL of a washing buffer (20 mM Tris-HCl, pH 7.6, 150 mM NaCl, 5 mM MgCl₂, 0.5% NP-40, 1 mM DTT). To each well, 40 µL of luciferase substrate (Promega, E151A) diluted 2,500-fold with the washing buffer was added, reaction was allowed for 5 minutes, and then luminescence was measured with a plate reader (PerkinElmer, 5103-35). The background (luminescence signal value obtained with adding the PPR protein and without adding RNA) was subtracted from the luminescences of the samples to which a solution containing each RNA and PPR protein was added, and the obtained values were used as the binding powers between the PPR protein and RNA.

The results are shown in Fig. 8. All of those prepared with the motif sequence v2 showed increase (1.3- to 3.6-fold) in binding power to the target sequence compared with those prepared with the motif sequence v1. In addition, 2 kinds of RNA probes having a non-target sequence (off target 1 and off target 2) (SEQ ID NOS: 66 and 69) were prepared, and binding of the proteins to them was examined. As a result, all of those prepared with v2 showed a higher target binding signal/non-target binding signal (S/N) ratio compared with those prepared with v1 (Fig. 8, upper left), and therefore it was found that v2 shows higher affinity and specificity to the target compared with v1.

### [Example 4: Detailed analysis of RNA binding performance of PPR proteins prepared with v2 motif]

### (Specificity evaluation)

By using the v2 motif, PPR proteins for 23 kinds of target sequences (T_1 to T_3, and T_5 to T_24, SEQ ID NOS: 46 to 48 and 51 to 69) were prepared (NL_v2_PPR1 to 3, and NL_v2_PPR5 to 24, SEQ ID NOS: 56 to 58 and 70 to 88), and RPB-ELISA was used to analyze bindings of all the combinations. The experimental method was the same as that used in Example 3.

The results are shown in Fig. 9 (upper part). It was found that the binding power to the target was strongest in the 21 kinds of PPR proteins, except for v2_17 and v2_24. These results indicate that PPR proteins can be stably prepared by using the v2 motif, and their binding specificity is high.

By using the V3.1 motif, PPR proteins for the same 23 kinds of target sequences were similarly prepared (SEQ ID NOS: 411 to 433 for the nucleotide sequences, and SEQ ID NOS: 434 to 456 for the amino acid sequences), and bindings of all the combinations were analyzed by using RPB-ELISA. The experimental method was the same as that used in Example 3.

The results are shown in Fig. 9 (lower part), and the table shown below. Those showing improved binding power compared with V2 were obtained.

**[Table 9]**

| Target No. | Binding acitivity(Target) | | v3.1/v2 | Target sequence | No. of base in seq. | | | |
|---|---|---|---|---|---|---|---|---|
| | v2 | v3.1 | | | A | U | G | C |
| 6 | 1.7.E+07 | 6.1.E+06 | 0.4 | CAACAUCAGUCUGAUAAG | 7 | 4 | 3 | 4 |
| 7 | 1.9.E+07 | 3.4.E+07 | 1.8 | CACAAUGUGGCCGAGGAC | 5 | 2 | 6 | 5 |
| 1 | 6.6.E+07 | 7.2.E+07 | 1.1 | GAAUGAACUCUUCCGGGA | 5 | 4 | 5 | 4 |
| 8 | 1.2.E+07 | 4.9.E+07 | 4.0 | AAGCCAGUUUUCAUUUUG | 4 | 8 | 3 | 3 |
| 9 | 4.7.E+07 | 2.2.E+06 | 0.0 | CACUAUUUAAGUUAUCAA | 7 | 7 | 1 | 3 |
| 10 | 7.8.E+06 | 5.8.E+07 | 7.5 | CAAACUUUCACUUUGAAA | 7 | 6 | 1 | 4 |
| 11 | 1.2.E+07 | 2.6.E+07 | 2.2 | GGUGGUGAGGCCCUGGGC | 1 | 3 | 10 | 4 |
| 12 | 3.8.E+07 | 1.8.E+07 | 0.5 | GACUCAGGAAUCGGCUCU | 4 | 4 | 5 | 5 |
| 13 | 2.3.E+06 | 4.0.E+07 | 17.5 | CAACAUCAAAGACACCAU | 9 | 2 | 1 | 6 |
| 14 | 2.5.E+07 | 2.5.E+07 | 1.0 | GUCAGAGGGUUCUGGAUU | 3 | 6 | 7 | 2 |
| 3 | 3.3.E+07 | 6.6.E+07 | 2.0 | CUGAGUCAUAACCAGCCU | 5 | 4 | 3 | 6 |
| 15 | 1.3.E+07 | 3.2.E+07 | 2.4 | GCAGAUAAUUAAUAAGAA | 10 | 4 | 3 | 1 |
| 16 | 2.8.E+07 | 6.3.E+07 | 2.2 | AAGGAUAAUAUCAAACAC | 10 | 3 | 2 | 3 |
| 17 | 2.4.E+07 | 3.3.E+07 | 1.4 | UUAUCAGACUGAUGUUGA | 5 | 7 | 4 | 2 |
| 18 | 1.1.E+07 | 2.9.E+07 | 2.8 | GGUUAGAGAUACAGUGUG | 5 | 5 | 7 | 1 |
| 19 | 1.3.E+07 | 1.3.E+07 | 1.0 | GUGGGGGUGGUAGGAAAU | 4 | 4 | 10 | 0 |
| 20 | 1.2.E+07 | 3.6.E+06 | 0.3 | GUGAUGUGGAGUUAAGGC | 4 | 5 | 8 | 1 |
| 5 | 6.0.E+07 | 5.6.E+07 | 0.9 | GGCAAAAAGAUCACUGUA | 8 | 3 | 4 | 3 |
| 2 | 5.7.E+07 | 4.4.E+07 | 0.8 | GAGAGGAAGCCUGAGAGU | 6 | 2 | 8 | 2 |
| 21 | 5.0.E+07 | 4.9.E+06 | 0.1 | GGAAGAGUGUCUGGAGCA | 5 | 3 | 8 | 2 |
| 22 | 5.6.E+07 | 5.5.E+07 | 1.0 | UGAUGAUGAUGAUGAUGA | 6 | 6 | 6 | 0 |
| 23 | 2.6.E+07 | 5.3.E+07 | 2.0 | UCUUUGCCAUUUCCCAUA | 3 | 8 | 1 | 6 |
| 24 | 3.3.E+07 | 5.5.E+07 | 1.7 | CCCAUAGAUGUGACAAGC | 6 | 3 | 4 | 5 |

The RNA binding performances of the PPR proteins shown in Fig. 9 are summarized in the tables shown below in terms of numerical values (log2 values).

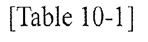

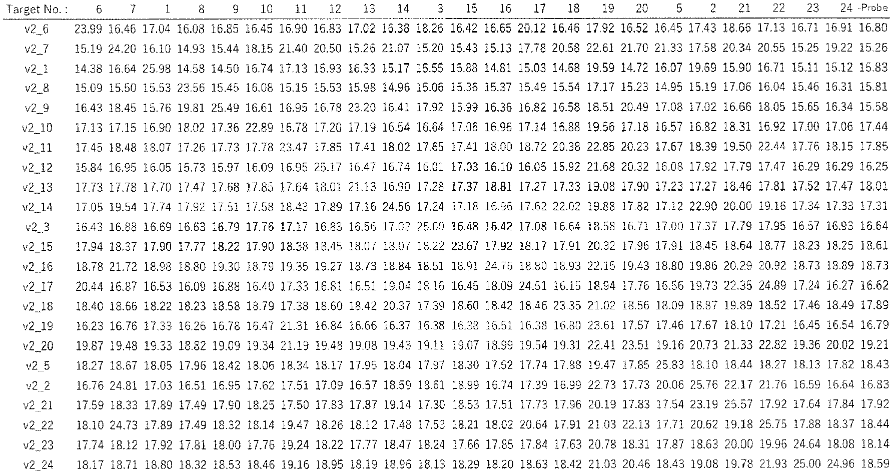

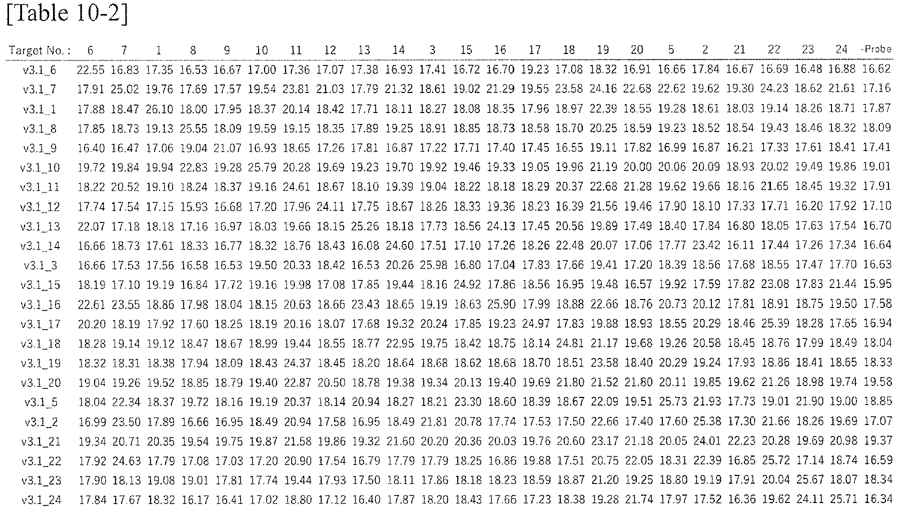

### (Affinity evaluation)

Further, EMSA was performed in order to calculate the affinity (Kd value) of each PPR protein to the target RNA thereof. *E. coli* expression plasmids were constructed for 10 kinds of PPR proteins among 23 kinds of those mentioned above, to which a streptavidin-binding peptide sequence was added on the N-terminus side, and a 6x His-tag sequence on the C-terminus side (SBP_v2_PPR1_HIS, SBP_v2_PPR2_HIS, SBP_v2_PPR3_HIS, SBP_v2_PPR6_HIS, SBP_v2_PPR9_HIS, SBP_v2_PPR12_HIS, SBP_v2_PPR15_HIS, SBP_v2_PPR16_HIS, SBP_v2_PPR20_HIS, and SBP_v2_PPR24_HIS, SEQ ID NOS: 89 to 97). The Rosetta (DE3) strain of *E. coli* was transformed with the plasmids, and cultured overnight at 37°C in 2 mL of the LB medium containing 100 µg/mL ampicillin. Then, 2 mL of the culture medium was transferred to 300 mL of the LB medium containing 100 µg/mL ampicillin, and culture was performed at 37°C until OD₆₀₀ reached 0.5 to 0.8. After the culture, the temperature of the medium was lowered to 15°C, then 0.1 mM IPTG was added, and culture was continued for further 12 hours. The culture medium was centrifuged at 5000 × g and 4°C for 10 minutes to collect the cells, 5 mL of a lysis buffer (20 mM Tris-HCl, pH 8.0, 150 mM NaCl, 0.5% NP-40, 1 mM DTT, 1 mM EDTA) was added, and the mixture was stirred on Voltex mixer, and sonicated to disrupt the cells. The disrupted mixture was centrifuged at 15,000 rpm and 4°C for 10 minutes, and the supernatant was collected.

Then, the objective proteins were purified by affinity chromatography using the SBP tag. Streptavidine Sepharose High Performance (GE Healthcare, 17511301) was taken in a volume of 100 µL, and the beads were collected by spin down, and equilibrated with a washing buffer (20 mM Tris-HCl, pH 8.0, 500 mM NaCl, 0.5% NP-40). The equilibrated beads were gently mixed with each of the previously collected cell extracts and permeabilized at 4°C for 10 minutes. The entire volume of the beads mixture was loaded on a column, and then the beads were washed with 10 mL of the washing buffer. Elution was performed with an elution buffer (20 mM Tris-HCl, pH 8.0, 500 mM NaCl, 2 mM biotin).

Then, affinity purification using the histidine tag was performed. First, 200 µL of Ni-NTA agarose (Qiagen, 30230) was collected, and after centrifugation, the beads were collected. To the beads, 100 µL of the washing buffer was added, and the beads were equilibrated by permeabilization at 4°C for 1 hour. The entire volume of the equilibrated beads was mixed with the protein solution eluted from the SBP beads, and reaction was allowed at 4°C for 1 hour. The beads were collected by centrifugation at 2,000 rpm for 2 minutes, and factors non-specifically binding to the beads were removed with 10 mL of a washing buffer (20 mM Tris-HCl, pH 8.0, 500 mM NaCl, 0.5% NP-40, 10 mM imidazole). Elution was performed with 60 µl of an elution buffer (20 mM Tris-HCl, pH 8.0, 500 mM NaCl, 0.5% NP-40, 500 mM imidazole). Purification degree was confirmed by SDS-PAGE, and the eluted solution was dialyzed overnight at 4°C against 20 mM Tris-HCl, pH 8.0, 150 mM NaCl, 0.5% NP-40, 1 mM DTT, 1 mM EDTA.

The total amount of the protein obtained after the dialysis was estimated by using Pierce 660nm Protein Assay Kit (Thermo fisher, 22662). To determine the amount of the objective protein, each dialyzed sample was subjected to SDS-PAGE on a 10% polyacrylamide gel, and CBB staining was performed. The image of the stained gel was captured with ChemiDoc Touch MP Imaging System (Biorad). The total band intensity and intensity of the objective band were obtained from the gel image. The amount of the objective protein was calculated by multiplying the total protein amount by the ratio of the objective band intensity to the total band intensity. This value was used to calculate the molar concentration of purified protein in the dialyzed sample. On the basis of the molar concentrations calculated above, diluted protein solutions of 400 nM, 200 nM, 100 nM, 50 nM, 20 nM, 10 nM, 5 nM, 2 nM and 1 nM were prepared. Dilution was performed with a binding buffer (20 mM Tris-HCl, pH 8.0, 150 mM NaCl, 0.5% NP-40, 1 mM DTT, 1 mM EDTA). The final concentrations of the 5'-end biotinylated RNA probes (RNA_1, RNA_2, RNA_3, RNA_6, RNA_9, RNA_12, RNA_15, RNA_16, RNA_20, and RNA_24) were adjusted to 20 nM with the binding buffer. RNA samples were heat-treated at 75°C for 1 minute, quenched and used for the following experiments.

The protein solution of each concentration prepared above was mixed with 20 nM RNA probe solution, and the binding reaction was allowed at 25°C for 20 minutes.

After the reaction, 2 µL of 80% glycerol was added, and the mixture was sufficiently suspended. Then, 10 µL of the mixture was applied to the ATTO 7.5% gel, and electrophoresis was performed at C.V. 150 V for 30 minutes.

The gels after the electrophoresis were transferred to the Hybond N+ membrane (GE, RPN203B). Then, RNA was UV cross-linked to the membrane by using ASTEC Dual UV Transilluminator UVA-15 (Astec, 49909-06). The membrane was blocked with a blocking buffer (6.7 mM NaH₂PO₄·2H₂O, 6.7 mM Na₂HPO₄·2H₂O, 125 mM NaCl, 5% SDS). In this operatopm, 0.5 µL of Stereptavidine-HRP (Abcam, ab7403) was added to the blocking buffer beforehand, and the antigen-antibody reaction was allowed with permeabilization for 15 minutes. The blocking buffer was discarded, and 20 mL of a washing buffer (0.67 mM NaH₂PO₄·2H₂O, 0.67 mM Na₂HPO₄·2H₂O, 12.5 mM NaCl, 0.5% SDS) was added to wash the membrane. This washing procedure was repeated five times, and then 20 ml of an equilibration buffer (100 mM Tris-HCl, pH 9.5, 100 mM NaCl, 10 mM MgCl₂) was added to permeabilize the membrane for 5 minutes. Then, Immunobilon Western Chemiluminescent HRP Substrate (Millipore, Cat. No. WBKLS0100) was added to the membrane, and bands were detected by using chemiluminescence of the biotinylated RNA. The gel images were captured by using ChemiDoc Touch MP Imaging System (Biorad). The band intensities of the unbound RNA probe band and the band shifted by binding to the protein were calculated. The equilibrium dissociation constant (Kd value) was calculated from the molar concentration of the protein and the ratio of the corresponding shifted band according to the Hill equation.

The results are shown in Fig. 10. It was found that the prepared PPR proteins had a Kd value of 10⁻⁹ to 10⁻⁷ M for the targets. The minimum value (high affinity) was 1.95 × 10⁻⁹, which is the lowest Kd value among those of the designed PPR proteins reported so far (see Table 1). It was also found that these Kd values correlated with the signal values obtained in the binding experiments based on RPB-ELISA (R2 = -0.85). On the basis of these results, it can be estimated that the Kd value is 10⁻⁶ to 10⁻⁷ M for the RPB-ELISA luminescence value of 1 to 2 × 10⁷, 10⁻⁷ to 10⁻⁸ M for the RPB-ELISA luminescence value of 2 to 4 × 10⁷, and ~10⁻⁸ or lower for the RPB-ELISA luminescence value larger than 4 × 10⁷.

### (Evaluation of successful construction rate)

Further, PPR proteins for 72 kinds of target sequences (T1 to T3, and T6 to T76, SEQ ID NOS: 46 to 48, 51 to 69, and 117 to 168) were prepared by using the v2 motif (NL_v2_PPR1 to 3, and NL_v2_PPR6 to 76, SEQ ID NOS: 56 to 58, 70 to 88, and 169 to 220), and the probability of successful construction was calculated by using RPB-ELISA. Biotinylated RNA probes containing the target sequence (RNA_1 to 3, and RNA_6 to 76, SEQ ID NOS: 61 to 63, 98 to 116, and 221 to 272) and a biotinylated RNA probe (RNA51, SEQ ID NO: 247) containing the non-target sequence (T_51, SEQ ID NO: 143) were prepared (Greinar). The experimental method was the same as that used in Example 3. The results are shown in Fig. 11.

Of the 72 kinds of the PPR proteins, 63 (88%) were estimated to have a Kd value of 10⁻⁶ M or lower (RPB-ELISA value of 1 × 10⁷ or higher), 57 (79%) were estimated to have a Kd value of 10⁻⁷ M or lower (RPB-ELISA value of 2 × 10⁷ or higher), and 43 (40%) were estimated to have a Kd value of 10⁻⁸ M or lower (RPB-ELISA value of 4 × 10⁷ or higher). A value obtained by dividing the target binding signal by the non-target binding signal (S/N) was used as a value for evaluating the specificity. Those that showed an S/N higher than 10 were 54 (75%), and those that showed an S/N higher than 100 were 23 (32%). These results indicate that sequence-specific RNA-binding proteins can be efficiently prepared by preparing PPR proteins using the v2 motif.

### (Evaluation of target binding activity in relation to the number of PPR motifs)

Analysis was performed for the relation of the number of PPR motifs and target-binding activity. There were determined 13 kinds of 18-base target sequences, and the 3 nucleotides or 6 nucleotides of the 5'-end side of each sequence were deleted to design 15-base target sequences (T_1a, T_49a, T_3a, T_14a, T_40a, T_12a, T_13a, T_2a, T_38a, T_37a_T_39a, T_56a, and T_68a, SEQ ID NOS, 273, 275, 277, 279, 281, 283, 285, 287, 289, 291, 293, 395, and 297), and 12-base target sequences (T_1b, T_49b, T_3b, T_14b, T_40b, T_12b, T_13b, T_2b, T_38b, T_37b T_39b, T_56b, and T_68b, SEQ ID NOS: 274, 276, 278, 280, 282, 284, 286, 288, 290, 292, 294, 296, and 298), respectively. The corresponding PPR proteins (those of 15 motifs were named PPRxa, and those of 12 motifs were named PPRxb) were prepared (NL_v2_PPR1, 1a, and 1b; NL_v2_PPR49, 49a, and 49b; NL_v2_PPR3, 3a, and 3b; NL_v2_PPR14, 14a, and 14b; NL_v2_PPR 40, 40a, and 40b; NL_v2_PPR12, 12a, and 12b; NL_v2_PPR13, 13a, and 13b; NL_v2_PPR2, 2a, and 2b; NL_v2_PPR38, 38a, and 38b; NL_v2_PPR37, 37a, and 37b; NL_v2_PPR39, 39a, and 39b; NL_v2_PPR56, 56a, and 56b; and NL_v2_PPR68, 68a, and 68b, SEQ ID NOS: 56, and 299 to 324). For analysis by RPB-ELISA, biotinylated RNA probes containing the target sequence (T_1, T_49, T_3, T_14, T_40, T_12, T_13, T_2, T_38, T_37, T_39, T_56, and T_68) and a biotinylated RNA probe (RNA 51, SEQ ID NO: 247) containing a non-target sequence (T_51, SEQ ID NO: 143) were prepared, and binding activities of the respective PPR proteins to the target (on target) and non-target (off target) were analyzed by RPB-ELISA.

The results for each target sequence are shown in Fig. 12A. The averages of the values for each of the 18-, 15-, and 12-motif proteins are plotted as a box-and-whisker diagram shown in Fig. 12B. A higher number of the motifs provided higher binding strength, and the 18-motif proteins were found to enable more stable preparation of proteins with higher binding strength in comparison of the 18-motif and 15-motif proteins.

### [Example 5: Artificial control of splicing using PPR proteins]

In order to demonstrate that PPR proteins can bind to a target RNA molecule in the cells and enable desired RNA manipulation, an experiment was performed by using a splicing reporter (Fig. 13A). The splicing reporter (RG6) has a genetic structure comprising exon 1, intron 1, exon 2, intron 2, exon 3, etc. (Orengo et al., 2006 NAR). Into intron 1, exon 2, and intron 2, intron 4, and intron 5 of chicken cTNT, and an artificially created alternative exon sequence were inserted. This reporter had two splicing forms, and the amount ratio of mRNAs with and without skip of exon 2 is about 1:1. RFP and GFP genes are encoded in exon 3. In this respect, the reading frame changes depending on the presence or absence of exon 2, so that RFP is expressed with mRNA in which exon 2 is skipped, and GFP is expressed with mRNA in which exon 2 is not skipped. It is known that the amounts of the splicing forms of this reporter are controlled by splicing factors that bind to the regions of intron 1, exon 2, and intron 2 (Orengo et al., 2006 NAR). Therefore, 18 nucleotides sequences were selected from the regions of intron 1, exon 2, and intron 2, and whether the splicing form of the RG-6 reporter could be changed by PPR proteins that bind to those sequences was examined.

Seven kinds of target sequences (T77 to T83, SEQ ID NOS: 325 to 330) were selected from the RG6 reporter. The PPR protein genes were designed with both v1 and v2 motifs (v1_PPRsp1 to 6, and v2_PPRsp1 to 6, SEQ ID NOS: 331 to 342). The protein genes were cloned into pcDNA3.1 so that proteins fused with a nuclear localization signal on the N-terminus side and a FLAG epitope tag sequence on the C-terminus side should be expressed (NLS_v1PPRsp1 to 6, and NLS_v2PPRsp1 to 6, SEQ ID NOS: 343 to 354). pcDNA3.1 has the CMV promoter and SV40 poly-A signal (terminator), and the PPR protein gene was inserted between them.

The HEK293T cells were inoculated at a density of 1 × 10⁶ cells in 10 cm dish containing 9 mL of DMEM, and 1 mL of FBS, and cultured in an environment of 37°C and 5% CO₂ for 2 days, and then the cells were collected. The collected cells were inoculated on a PLL-coated 96-well plate at a density of 4 × 10⁴ cells/well, and cultured in an environment of 37°C and 5% CO₂ for 1 day. A mixture of 100 ng of PPR expression plasmid DNA, 100 ng of RG-6, 0.6 µL of Fugene (registered trademark)-HD (Promega, E2311), and 200 µL Opti-MEM was prepared, the whole volume thereof was added to the wells, and culture was performed in an environment of 37°C and 5% CO₂ for 2 days. As a control, a sample not containing any PPR expression plasmid DNA was also prepared. After the culture, GFP and RFP fluorescence images of each well were obtained by using a fluorescence microscope DMi8 (Leica). As for the imaging conditions, exposure time and gain at which the intensities of GFP and RFP became substantially the same were first determined by using a sample containing only the RG-6 plasmid, and the fluorescence images of the samples were obtained under the same conditions.

After acquisition of the image, total RNA was extracted by using the Maxwell (registered trademark) RSC simplyRNA Cells Kit. To a 0.2-mL tube, 500 ng of the extracted total RNA, 0.5 µL of 100 µM dT20 primer, and 0.5 µL of 10 mM dNTPs were added, left at 65°C for 5 minutes, and immediately cooled on ice. To this, 2 µL of 5x RT-buffer (Invitrogen, 18080-051), 0.5 µL of 0.1M DTT, 0.5 µL of 40U/µL RNaseOUT (Invitrogen, 18080-051), and 0.5 µL of 200 unit/uL SupperScript III (Invitrogen, 18080-051) were added, and reaction was allowed in a thermal cycler at 50°C for 50 minutes, then at 85°C for 5 minutes, and cooled to 16°C. The reverse transcribed sample was diluted 10-fold with sterile water. To a 0.2-mL tube, 2 µL of the reaction mixture, 10 µL of 5× GXL buffer (TAKARA, R050A), 4 µL of 2.5 mM dNTPs, 1.5 µL of 10 µM RT-Fw primer (5'-CAAAGTGGAGGACCCAGTACC-3', SEQ ID NO: 355), 1.5 µL of 10 µM RT-Rv Primer (5'-GCGCATGAACTCCTTGATGAC-3', SEQ ID NO: 356), 1 µL of GXL (TAKARA, R050A), and 31.5 µL of sterile water were added, reaction was allowed in a thermal cycler at 98°C for 2 minutes, followed by 35 cycles of 98°C for 10 seconds, 58°C for 15 seconds, and 68°C for 5 seconds, and then the reaction mixture was cooled to 12°C. The reaction mixture was diluted 10 times, and electrophoresed with MultiNA (SHIMADZU, MCE202). The band of about 114 bp and the band of about 142 bp were regarded as the band of exon-skipped RNA and the band of unskipped RNA, respectively, and the band intensities of the samples were calculated. A value obtained by dividing the 114 bp band intensity by the sum of the 114 bp band intensity and the 142 bp band intensity was defined as the splicing ratio.

The results are shown in Figs. 13B and 13C. It was found that the splicing ratio was 0.48 when only the RG6 reporter was introduced, and was similar when PPRsp4 was introduced, but significantly changed when the other PPRs were introduced. In comparison of v1 and v2, v2 provided a larger change except for PPRsp4. These splicing ratios were also consistent with the RFP and GFP expression ratios shown in Fig. 13B. These results verified that PPR proteins can be used to change exon skipping, and revealed that the v2 motif can be used to change splicing even more efficiently.

### [Example 6: Regulation of aggregation of PPR protein]

A PPR protein using V2 motif (SEQ ID NO: 457 for nucleotide sequence, and SEQ ID NO: 458 for amino acid sequence) and a PPR protein using v3.2 motif (SEQ ID NO: 459 for nucleotide sequence, and SEQ ID NO: 460 for amino acid sequence) were prepared in an *E. coli* expression system, respectively, purified, and separated by gel filtration chromatography.

### (Expression and purification of proteins)

The *E. coli* Rosetta strain was transformed with pE-SUMOpro Kan plasmid containing a DNA sequence encoding the objective PPR protein, and cultured at 37°C, then the temperature was lowered to 20°C when OD₆₀₀ reached 0.6, and IPTG was added at a final concentration of 0.5 mM so that the objective PPR was expressed in the *E. coli* cells as SUMO-fused protein. The cells were cultured overnight, then collected by centrifugation, and resuspended in a lysis buffer (50 mM Tris-HCl, pH 8.0, 500mM NaCl). The *E. coli* cells were disrupted by sonication, and centrifuged at 17,000g for 30 minutes, then the supernatant fraction was applied to an Ni-Agarose column, the column was washed with the lysis buffer containing 20 mM imidazole, and then the SUMO-fused objective PPR protein was eluted with the lysis buffer containing 400 mM imidazole. After the elution, the SUMO protein was cleaved from the objective PPR protein with Ulp1, and at the same time, the protein solution was substituted with an ion-exchange buffer (50 mM Tris-HCl, pH 8.0, 200 mM NaCl) by dialysis. Subsequently, cation exchange chromatography was performed by using SP column. After application to the column, proteins were eluted with gradually increasing NaCl concentration of from 200 mM to 1 M. The fraction containing the objective PPR protein was subjected to final purification by gel filtration chromatography using Superdex 200 column. The objective PPR protein eluted from the ion exchange column was applied to the gel filtration column equilibrated with a gel filtration buffer (25 mM HEPES, pH 7.5, 200 mM NaCl, 0.5 mM tris(2-carboxyethyl)phosphine (TCEP)). Finally, the fraction containing the objective PPR protein was concentrated, frozen in liquid nitrogen, and stored at -80°C until used for the next analysis.

### (Gel filtration chromatography)

The purified recombinant PPR protein was prepared at a concentration of 1 mg/ml. For gel filtration chromatography, Superdex 200 increase 10/300 GL (GE Healthcare) was used. To the gel filtration column equilibrated with 25 mM HEPES pH7.5, 200 mM NaCl, 0.5 mM tris(2-carboxyethyl)phosphine (TCEP), the prepared protein was applied, and the absorbance of the solution eluted from the gel filtration column was measured at 280 nm to analyze the properties of the protein.

### (Results)

The results are shown in Fig. 14. The smaller volume of the elution fraction (Elution vol.) means a.larger molecular size. The protein using v2 were eluted in elution fractions of 8 to 10 mL, whereas the peak of the protein using v3.2 was observed in elution fractions of 12 to 14 mL. This result suggested the possibility that the protein using v2 aggregated due to the larger protein size thereof, and the aggregation was improved in the protein using v3.2.

## Claims

1. A PPR motif, which is any one of the following PPR motifs:
(A-1) a PPR motif consisting of the sequence of SEQ ID NO: 9, or a PPR motif consisting of the sequence of SEQ ID NO: 9 having a substitution selected from the group consisting of substitution of the amino acid at position 10 with tyrosine, substitution of the amino acid at position 15 with lysine, substitution of the amino acid at position 16 with leucine, substitution of the amino acid at position 17 with glutamic acid, substitution of the amino acid at position 18 with aspartic acid, and substitution of the amino acid at position 28 with glutamic acid, or a PPR motif consisting of the sequence of SEQ ID NO: 401 or a PPR motif consisting of the sequence of SEQ ID NO: 401 having a substitution selected from the group consisting of substitution of the amino acid at position 10 with tyrosine, substitution of the amino acid at position 16 with leucine, substitution of the amino acid at position 17 with glutamic acid, substitution of the amino acid at position 18 with aspartic acid, and substitution of the amino acid at position 28 with glutamic acid;
(A-2) a PPR motif consisting of the sequence of SEQ ID NO: 9 or 401 having a substitution, deletion, or addition of 1 to 20 amino acids other than the amino acids at positions 1, 2, 3, 4, 6, 7, 9, 11, 12, 14, 19, 26, 30, 33, and 34, and having an adenine-binding property;
(A-3) a PPR motif having a sequence identity of at least 42% to the sequence of SEQ ID NO: 9 or 401, provided that the amino acids at positions 1, 2, 3, 4, 6, 7, 9, 11, 12, 14, 19, 26, 30, 33, and 34 are identical, and having an adenine-binding property;
(C-1) a PPR motif consisting of the sequence of SEQ ID NO: 10, or a PPR motif consisting of the sequence of SEQ ID NO: 10 having a substitution of amino acid selected from the group consisting of substitution of the amino acid at position 2 with serine, substitution of the amino acid at position 5 with isoleucine, substitution of the amino acid at position 7 with leucine, substitution of the amino acid at position 8 with lysine, substitution of the amino acid at position 10 with phenylalanine or tyrosine, substitution of the amino acid at position 15 with arginine, substitution of the amino acid at position 22 with valine, substitution of the amino acid at position 24 with arginine, substitution of the amino acid at position 27 with leucine, and substitution of the amino acid at position 29 with arginine;
(C-2) a PPR motif consisting of the sequence of SEQ ID NO: 10 having a substitution, deletion, or addition of 1 to 25 amino acids other than the amino acids at positions 1, 3, 4, 14, 18, 19, 26, 30, 33, and 34, and having a cytosine-binding property;
(C-3) a PPR motif having a sequence identity of at least 25% to the sequence of SEQ ID NO: 10, provided that the amino acids at positions 1, 3, 4, 14, 18, 19, 26, 30, 33, and 34 are identical, and having a cytosine-binding property;
(G-1) a PPR motif consisting of the sequence of SEQ ID NO: 11, or a PPR motif consisting of the sequence of SEQ ID NO: 11 having a substitution selected from the group consisting of substitution of the amino acid at position 10 with phenylalanine, substitution of the amino acid at position 15 with aspartic acid, substitution of the amino acid at position 27 with valine, substitution of the amino acid at position 28 with serine, and substitution of the amino acid at position 35 with isoleucine;
(G-2) a PPR motif consisting of the sequence of SEQ ID NO: 11 having a substitution, deletion, or addition of 1 to 21 amino acids other than the amino acids at positions 1, 2, 3, 4, 6, 7, 9, 14, 18, 19, 26, 30, 33, and 34, and having a guanine-binding property;
(G-3) a PPR motif having a sequence identity of at least 40% to the sequence of SEQ ID NO: 11, provided that the amino acids at positions 1, 2, 3, 4, 6, 7, 9, 14, 18, 19, 26, 30, 33, and 34 are identical, and having a guanine-binding property;
(U-1) a PPR motif consisting of the sequence of SEQ ID NO: 12, or a PPR motif consisting of the sequence of SEQ ID NO: 12 having a substitution selected from the group consisting of substitution of the amino acid at position 10 with phenylalanine, substitution of the amino acid at position 13 with serine, substitution of the amino acid at position 15 with lysine, substitution of the amino acid at position 17 with glutamic acid, substitution of the amino acid at position 20 with leucine, substitution of the amino acid at position 21 with lysine, substitution of the amino acid at position 23 with phenylalanine, substitution of the amino acid at position 24 with aspartic acid, substitution of the amino acid at position 27 with lysine, substitution of the amino acid at position 28 with lysine, substitution of the amino acid at position 29 with arginine, and substitution of the amino acid at position 31 with leucine;
(U-2) a PPR motif consisting of the sequence of SEQ ID NO: 12 having a substitution, deletion, or addition of 1 to 22 amino acids other than the amino acids at positions 1, 2, 3, 4, 6, 11, 12, 14, 19, 26, 30, 33, and 34, and having a uracil-binding property; and
(U-3) a PPR motif having a sequence identity of at least 37% to the sequence of SEQ ID NO: 12, provided that the amino acids at positions 1, 2, 3, 4, 6, 11, 12, 14, 19, 26, 30, 33, and 34 are identical, and having a uracil-binding property.

2. Use of the PPR motif according to claim 1 for preparation of a PPR protein of which target RNA has a length of 15 bases or longer.

3. Use of the PPR motif according to claim 1 for preparation of a PPR protein, which is for enhancing binding performance of the PPR protein to a target RNA.

4. A PPR protein comprising n of PPR motifs and capable of binding to a target RNA consisting of a sequence of n bases in length, wherein:
the PPR motif for adenine in the base sequence is the PPR motif of (A-1), (A-2), or (A-3) defined in claim 1;
the PPR motif for cytosine in the base sequence is the PPR motif of (C-1), (C-2), or (c-3) defined in claim 1;
the PPR motif for guanine in the base sequence is the PPR motif of (G-1), (G-2), or (G-3) defined in claim 1; and
the PPR motif for uracil in the base sequence is the PPR motif of (U-1), (U-2), or (U-3) defined in claim 1.

5. The protein according to claim 4, wherein n is 15 or larger.

6. The protein according to claim 4 or 5, wherein the first PPR motif from the N-terminus is any one of the following motifs:
(1st_A-1) a PPR motif consisting of the sequence of SEQ ID NO: 402 having such substitutions of the amino acids at positions 6 and 9 that any one of the combinations defined below is satisfied;
(1st_A-2) a PPR motif comprising the sequence of (1st_A-1) having a substitution, deletion, or addition of 1 to 9 amino acids other than the amino acids at positions 1, 4, 6, 9, and 34, and having an adenine-binding property;
(1st_A-3) a PPR motif having a sequence identity of at least 80% to the sequence of (1st_A-1), provided that the amino acids at positions 1, 4, 6, 9, and 34 are identical, and having an adenine-binding property;
(1st_C-1) a PPR motif consisting of the sequence of SEQ ID NO: 403;
(1st_C-2) a PPR motif comprising the sequence of (1st_C-1) having a substitution, deletion, or addition of 1 to 9 amino acids other than the amino acids at positions 1, 4, 6, 9, and 34, and having a cytosine-binding property;
(1st_C-3) a PPR motif having a sequence identity of at least 80% to the sequence of (1st_C-1), provided that the amino acids at positions 1, 4, 6, 9, and 34 are identical, and having a cytosine binding property;
(1st_G-1) a PPR motif consisting of the sequence of SEQ ID NO: 404 having such substitutions of the amino acids at positions 6 and 9 that any one of the combinations defined below is satisfied;
(1st_G-2) a PPR motif comprising the sequence of (1st_G-1) having a substitution, deletion, or addition of 1 to 9 amino acids other than the amino acids at positions 1, 4, 6, 9, and 34, and having a guanine-binding property;
(1st_G-3) a PPR motif having a sequence identity of at least 80% to the sequence of (1st_G-1), provided that the amino acids at positions 1, 4, 6, 9, and 34 are identical, and having a guanine-binding property;
(1st_U-1) a PPR motif consisting of the sequence of SEQ ID NO: 405 having such substitutions of the amino acids at positions 6 and 9 that any one of the combinations defined below is satisfied;
(1st_U-2) a PPR motif comprising the sequence of (1st_U-1) having a substitution, deletion, or addition of 1 to 9 amino acids other than the amino acids at positions 1, 4, 6, 9, and 34, and having a uracil-binding property; and
(1st_U-3) a PPR motif having a sequence identity of at least 80% to the sequence of (1st_U-1), provided that the amino acids at positions 1, 4, 6, 9, and 34 are identical, and having a uracil-binding property:
- a combination of asparagine as the amino acid at position 6 and glutamic acid as the amino acid at position 9,
- a combination of asparagine as the amino acid at position 6 and glutamine as the amino acid at position 9,
- a combination of asparagine as the amino acid at position 6 and lysine as the amino acid at position 9, and
- a combination of aspartic acid as the amino acid at position 6 and glycine as the amino acid at position 9.

7. A method for controlling RNA splicing, which uses the protein according to any one of claims 4 to 6.

8. A method for detecting RNA, which uses the protein according to any one of claims 4 to 6.

9. A fusion protein of at least one selected from the group consisting of a fluorescent protein, a nuclear localization signal peptide, and a tag protein, and the protein according to any one of claims 4 to 6.

10. A nucleic acid encoding the PPR motif according to claim 1, or the protein according to any one of claims 4 to 6.

11. A vector comprising the nucleic acid according to claim 10.

12. A cell (except for human individual) containing the vector according to claim 11.

13. A method for manipulating RNA, which uses the PPR motif according to claim 1, the protein according to any one of claims 4 to 6, or the vector according to claim 11 (implementation in human individual is excluded).

14. A method for producing an organism, which comprises the manipulation method according to claim 13.
